# EUROPEAN PATENT APPLICATION

(11) **EP 3 195 872 A1**
(43) Date of publication of application: **26.07.2017**
(21) Application number: 15890050.6
(22) Date of filing: 28.10.2015
(51) Int. Cl.: A61K 36/899, A61K 31/352, A61P 31/12, A61P 37/00, A61P 39/06

(54) **PLANT-BASED BIOLOGICALLY ACTIVE SUBSTANCE HAVING A POLYPHARMACOLOGICAL EFFECT**

(30) Priority: 24.04.2015 UA 201503915
(71) Applicant: Atamaniuk, Victor, Kyivo-Svyatoshinsky raion, Kyivskaya obl. 08150 (US); Novik, Anatolii, Kyiv 04053 (UA)
(72) Inventor: ATAMANIUK, Victor, Boyarka (UA)
(74) Representative: Benatov, Samuil Gabriel
(86) International application number: PCT/UA2015/000101
(87) International publication number: WO 2016/171640

(57) **Abstract**

The invention relates to pharmacology and may be used for producing medicinal agents for treating and preventing viral diseases caused by the herpes, influenza and hepatitis B and C viruses, and also virus-induced immunodeficiencies. A biologically active substance having a polypharmacological effect is made from the green parts and spikelets of cereals of the family Gramineae, genus Calamagrostis Adans and/or genus Deschampsia Beauv, and contains flavonoids, specifically aglycones of the flavonoids tricin, apigenin, luteolin, quercetin and rhamnazin, and/or flavonoid glycosides of tricin, apigenin, luteolin, quercetin, and rhamnazin, and excipients, and has the following composition by mass percent: tricin flavonoid aglycone and/or tricin flavonoid glycosides: 0.016-2.062%; apigenin flavonoid aglycone and/or apigenin flavonoid glycosides: 0.010-1.393%; luteolin flavonoid aglycone and/or luteolin flavonoid glycosides: 0.01-4.979%; quercetin flavonoid aglycone and/or quercetin flavonoid glycosides: 0.001-0.771 %; rhamnazin flavonoid aglycone and/or rhamnazin flavonoid glycosides: 0.104-0.203%; excipients: 99.868-90.592%. In this way, the biologically active substance is perfected by determining the specific compositions of the active ingredients thereof, and the physical, chemical and biological characteristics thereof, resulting in the invention of an optimal composition (Fig. 1) for achieving an antiviral effect with regard to specific viruses, and dosages when creating medicinal forms. In addition, it has been determined that the biologically active substance is an inducer of a type-γ endogenous interferon, displays an apoptosis-modulating effect, has antioxidant properties and enhances cell resistance to free radical stress. The antiviral effect with regard to specific viruses has been established to be an antiviral effect with regard to the type 2 herpes simplex virus, the influenza virus, and the bovine viral diarrhea virus (hepatitis C virus).

## Description

### FIELD

The invention relates to medicine, more specifically to pharmacology and to the composition of the biologically active substance (BAS) having a polypharmacological effect made of herbal substances comprising compounds of flavonoid aglycones, flavonoid glycosides and excipients. The BAS may be used to produce medicinal forms for treating and preventing viral diseases caused by the herpes, influenza and hepatitis B and C viruses, and also virus-induced immunodeficiencies.

### PRIOR ART

During the last years more attention is paid to antiviral agents made of plant raw materials with identified substances affecting directly the viral agent. Such flavonoid as tricin (5,7,4-trihydroxy-3',5'-dimethoxyflavone) is a known molecule having a broad antiviral effect.

It is known that tricin is effective against the influenza virus, the cytomegalovirus and the herpes simplex virus (Japanese patent Nº JP 2010254649 (A), published on Nov. 11, 2010; Japanese patent Nº JP 2006265248 (A), published on Oct. 05, 2006). It was established that the concentration of tricin obtained from Sasa albo-marginata, which inhibits development of CMV infection on the cellular level (antiviral effect) for 50%, is 0.42 mcm (or 0.139 mcg/ml) (Antiviral Research 91 (2011), p. 296-303), and an antiviral effect of synthetic tricin on the influenza virus type A(H1N1) is observed at concentrations equal to 3.3 mcm (or 1.09 mcg/ml) or above this value (Antiviral Chemistry and Chemotherapy 2011, 22: 1-11; Joi 103851 / MP 1782).

Chemically pure aglycones of tricin or complex tricin compounds in combination with compounds of apigenin and/or luteolin, and/or quercetin are obtained from plant raw materials: bamboo leaves, Sasa, rice leafs. Chemically pure flavonoid compounds oxidize quickly and are hydrophobic, so their use is limited to the sphere of scientific research at the cellular level. (Microbes and Infection. 2012 p. V.14. Is. 12. p 1086-1092). Complex compounds such as glycosides of tricin, apigenin, luteolin, quercetin aglycones and their complexes are hydrophilic; and, at the level of the whole organism, the synergistic effect and increased bioavailability is peculiar for complexes of flavonoid compounds of different molecules compared with the effect of certain pure substances. (In vivo 2011, 25 (5), 757-762, Journal of Food, Vol. 13, No. 1, p.140-150).

A biologically active substance (BAS) from plant raw material for the treatment and prevention of pathological conditions produced by the method disclosed in Ukrainian patent Nº UA 54362 (C2), A61K 35/78, dated Feb. 17, 2003, is the most similar to the claimed invention with regard to chemical composition and origin. A mixture of plants of the genus Calamagrostis Adans and genus Deschampsia Beauv, collected in the period after the stalks are sprung and before the end of spikelets flowering, are used as a plant raw material.

Such BAS has quite low toxicity, provides a wide range of pharmacological effects on the human organism, however the specific composition of the active agents is not determined, the effect against specific viruses is not defined, and recommended dosages for producing pharmaceutical forms are not provided.

The task of this invention is to improve the BAS by determining specific compositions of BAS active ingredients, their physical, chemical, and biological properties, to determine its optimal composition for an antiviral effect on specific viruses, dosages for producing pharmaceutical forms with retention of the low toxicity of the BAS.

### SUMMARY OF THE INVENTION

The task set above is solved with the help of the biologically active substance having a polypharmacological effect which is made from the green parts and spikelets of cereals of the family Gramineae, genus Calamagrostis Adans and/or genus Deschampsia Beauv, and contains flavonoids, specifically aglycones of tricin, apigenin, luteolin, quercetin and rhamnazin flavonoids and/or flavonoid glycosides of tricin, apigenin, luteolin, quercetin, and rhamnazin, and excipients, and has the following composition by mass percent:
tricin flavonoid aglycone and/or tricin flavonoid glycosides: 0.016-2.062%;
apigenin flavonoid aglycone and/or apigenin flavonoid glycosides: 0.010-1.393%;
luteolin flavonoid aglycone and/or luteolin flavonoid glycosides: 0.01-4.979%;
quercetin flavonoid aglycone and/or quercetin flavonoid glycosides: 0.001-0.771%;
rhamnazin flavonoid aglycone and/or rhamnazin flavonoid glycosides: 0.104-0.203%;
excipients: 99.868-90.592%.

More preferably, the biologically active substance contains hydrocarbon compounds, amino acids, chlorophylls as excipients.

More preferably, the biologically active substance is made from wood small-reed (Calamagrostis epigejos L. genus Calamagrostis Adans) and/or tufted hairgrass (Deschampsia cespitosa L. genus Deschampsia Beauv).

More preferably, the content of O-glycoside forms of flavonoids is (53.545÷86.422)% and the content of C-glycoside form of flavonoids is (43.293÷4.910)% of total flavonoid compounds in the biologically active substance, the rest are flavonoid aglycones.

To establish a cause-and-effect relationships between the specific active constituents and properties of the BAS, its optimal composition, studies were carried out using standard techniques (V.A. Mironov, S. Yankovskiy. Spektroskopiya v organicheskoy khimii [Spectroscopy in Organic Chemistry]. Moscow. Chemistry. 1985 - 230 p. (in Russian) K.S. Senchev. Prakticheskoe rukovodstvo po zhydkostnoy khromatografii [Practical guide to liquid chromatography]. Moscow. Technosphera, 2010 - 272 p. (in Russian)).

When flavonoid aglycones are in the form of O- and/or C-glycosides, the solubility of biologically active substances in aqueous media increases and the synergistic effect and bioavailability of the mixture of flavonoid glycosides are ensured at the level of the whole organism.

Five batches of model extracts were made from herbs gathered in different seasons. A batch of model extracts consisted of crude extracts: mixtures of herbs Calamagrostis epigejos and Deschampsia cespitosa at the ratio 1:1, and from each herb individually at a weight ratio of raw material to ethyl alcohol in the range from 1:10 to 3:1. Five batches of extracts were used to determine the limits of fluctuations range for the content of studied substances and to determine their average values. Model extracts were prepared with the help of known extraction methods, namely: maceration, percolation, ultrasonic extraction and combination of these methods (Innovatsionnye tekhnologii I oborudovanie farmatsevticheskogo proizvodstva [Innovative technologies and equipment for pharmaceutical production] (edited by N.V. Menshutina) Volume 2 (pp. 33-88), Moscow: Binom. 2013 - 480 p). During the extraction process, the solid residue index was periodically monitored. For extracts with a weight ratio of raw material to alcohol equal to 1:10 extraction process was terminated when the solid residue index was 0.5% (the average value of density is 0.8010 g/ml). For extracts with a weight ratio of raw material to alcohol equal to 1:2 extraction process was terminated when the solid residue index value was 1.2% (the average value of density is 0.8100 g/ml). And for extracts with a weight ratio of raw material to alcohol equal to 3:1 extraction process was terminated when the solid residue index was 2.5% (the average value of density is 0.8250 g/ml).

At first, the composition of the flavonoid aglycones, flavonoid glycosides was determined in the crude extract, which was produced at the ratio of raw material to alcohol equal to 1:2.

BAS composition was determined with the methods of instrumental analytical physical chemistry as follows: high-performance liquid chromatography-diode array detector method (HPLC, UPLC-PDA); gas chromatography-mass spectrometry - electron impact ionization (GC-MS); ultra-performance liquid chromatography-electrospray ionization tandem mass spectrometry (UPLC-MS/MS); nuclear magnetic resonance (NMR).

After identification and determination of substances in the crude extract, qualitative and quantitative content of the BAS in model extracts, prepared from each herb separately, was determined.

To determine the specific effect, a comparative study of the BAS crude extract and extracts prepared from each herb separately was conducted.

The BAS is present in plants in trace amounts compared to the macro components: chlorophylls, plant fiber. Therefore, the first step when separating biologically active substances is to remove the macro-components. A chlorophyll (liposoluble) fraction, which is hydrophobic, is removed with the help of an organic solvent, for example hexane or chloroform.

Hydrocarbons in plant raw materials are presented as the mono- and polysaccharides both in a free state and in combination with aglycones, which have a hydroxyl group. Free hydrocarbons that show hydrophilic properties are removed by extraction, i.e. aglycones are extracted and the hydrocarbons remain in the aqueous phase. Taking into account that flavonoid aglycones are present in plants both in a free state and in a glycosidic form, therefore, after the removal of chlorophyll fraction, in order to conduct qualitative and quantitative determination all aglycones (free and glycosidic ones) must be changed into one analytical form - aglycones free of hydrocarbon fragments. This is achieved with the help of acid hydrolysis: hydrocarbon fragments are separated under the action of hydrogen ions to glycosidic forms of flavonoids.

The chemical structure of flavonol and flavone glycoside compounds was determined by examining the products of hydrolysis with spectrophotometric, chromatographic methods (HPLC, GC with different detectors).

### BRIEF DESCRIPTION OF FIGURES

The scope of the invention is illustrated with the help of figures:
Fig. 1. HPLC, UPLC-PDA chromatogram of the crude extract and the absorption spectra of the isolated compounds.
Fig. 2. HPLC, UPLC-PDA chromatogram of the crude extract after hydrolysis and absorption spectra of the isolated compounds
Fig. 3. HPLC, UPLC-PDA chromatogram of preparatively isolated compounds of dominant aglycone - tricin - and its absorption spectra.
Fig. 4. GC-MS chromatogram of TMS derivatives of compounds of dominant aglycone - tricin (ion composition spectrum).
Fig. 5. GC-MS chromatogram of TMS derivatives of compounds of dominant aglycone - tricin (retention time of ion with mass of 531.00).
Fig. 6. ¹H-NMR spectrum of tricin obtained from the crude extract.
Fig. 7. ¹³C-NMR spectrum of tricin obtained from the crude extract.
Fig. 8. BAS dominant aglycones and their names.
Fig. 9. Formation scheme for O-glycosides and C-glycosides.
Fig. 10. Calibration curves showing how mass peak area 271 depends on concentration.
Fig. 11. Calibration curve showing how mass peak area 331 depends on concentration.
Fig. 12. Calibration curve showing how mass peak area 415÷416 depends on concentration.
Fig. 13. Calibration curve showing how mass peak area 465 depends on concentration.
Fig. 14. Influence of hydrogen ion concentration (number of moles of HCl) on hyperoside and vitexin resistance in case of solution heating in boiling water bath for 120 minutes.
Fig. 15. UPLC-MS/MS. Where:
   1 - PDA chromatogram for the crude extract;
   2 - total mass-chromatogram in case of scanning according to the mass of individual ions;
Fig. 16. UPLC-MS/MS. Where:
   3 - scanning by mass 331 with determination of chromatographic peak areas;
   4 - chromatogram for scanning by mass 331.
Fig. 17. UPLC-MS/MS. Where:
   1- PDA chromatogram for the crude extract after hydrolysis;
   2- total mass-chromatogram in case of scanning according to the mass of individual ions;
Fig. 18. UPLC-MS/MS. Where:
   3 - scanning by mass 331 with determination of chromatographic peak areas;
   4 - chromatogram for scanning by mass 331.
Fig. 19. HPLC, UPLC-PDA chromotography and absorption spectra of chromatographic peak of ethanol extract of Calamagrostis epigejos.
Fig. 20. HPLC, UPLC-PDA chromotography and absorption spectra of chromatographic peak of ethanol extract of Decshampsia cespitosa.
Fig. 21. The effect of the BAS on Jurkat cell growth.
Fig. 22. Content of hypodiploid cells (%) in Jurkat culture after incubation with the BAS.
Fig. 23. Content of hypodiploid cells in Jurkat culture after incubation with the BAS during two days and with subsequent induction of apoptosis by Vepesid.
Fig. 24. The effect of the BAS on generation of superoxide anion radical by cells MT-4.
Fig. 25. The effect of the BAS on generation of superoxide anion radical by cells Namalwa.
Fig. 26. The effect of the BAS on generation of superoxide anion radical by homogenate of cells MT-4.
Fig. 27. The effect of the BAS on chemoluminescence of the cells MT-4 induced by H₂O₂.

Due to the great number of graphic material and for the better understanding of the idea the names of figures are also specified below the latter.

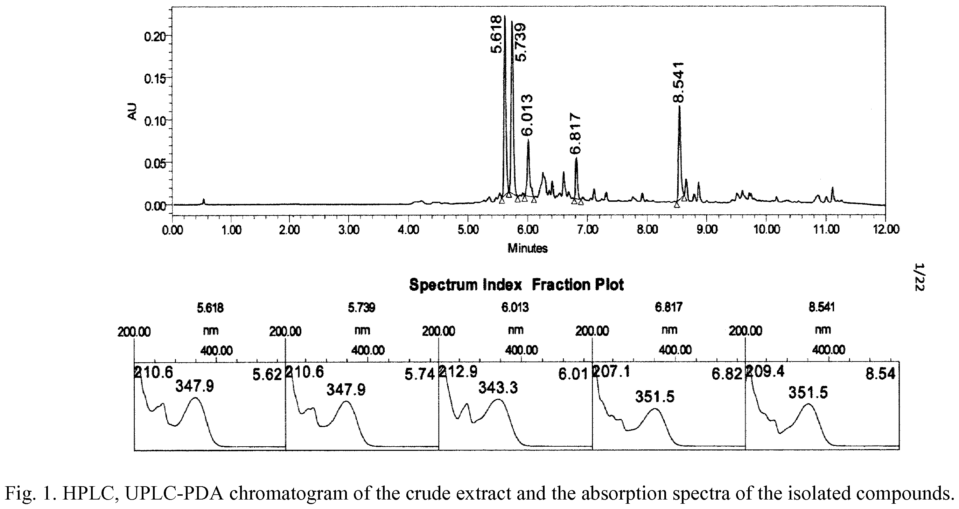

A typical chromatogram of compounds for the crude extract (the weight ratio of raw material to ethyl alcohol is 1:2) diluted with 96% ethanol at the ratio 1:1 and obtained by HPLC with diode-array detector is shown in Fig. 1. The analysis is performed using a chromatograph Waters ACQUITY UPLC BEH C₁₈ Column (2.1 x 150 mm, 1.7 µm) under the following conditions: injection volume is 1 µl, thermostat temperature is 30°C, mobile phase velocity is 0.3 µl /min, detection at 350 nm. The mobile phase is (water: acetonitrile) = (60:40) in a gradient mode, water at pH 2.2 (phosphoric acid). Figure 1 also shows the absorption spectra of the chromatographically separated crude extract compounds (Spectrum Index Fraction Plot).

The absorption spectra of the chromatographically separated compounds (Fig. 1) with a retention time ranging from 5.0 minutes (min.) to 10 minutes indicate that there is a set of compounds of one class in the solution - flavonoids, which have the same "backbone" of the molecule. The difference in the spectra is explained by the nature of the substituents in the molecule composition. Molecules are identified using acidic hydrolysis, which in case of O-glycosides enables to isolate (release) aglycones. Acid hydrolysis was carried out as follows: hydrochloric acid is added to the extract to obtain a solution with pH equal to approximately 2 (in relation to 2 Mol hydrochloric acid), a flask with the solution, coupled to a reflux condenser, is incubated in water bath for 2 hours during boiling, then it is cooled, and hydrolyzed aglycones and glycosidic forms are isolated with ethyl acetate, the organic solvent is removed under vacuum and the residue is dissolved in ethanol (9:1) and analyzed.

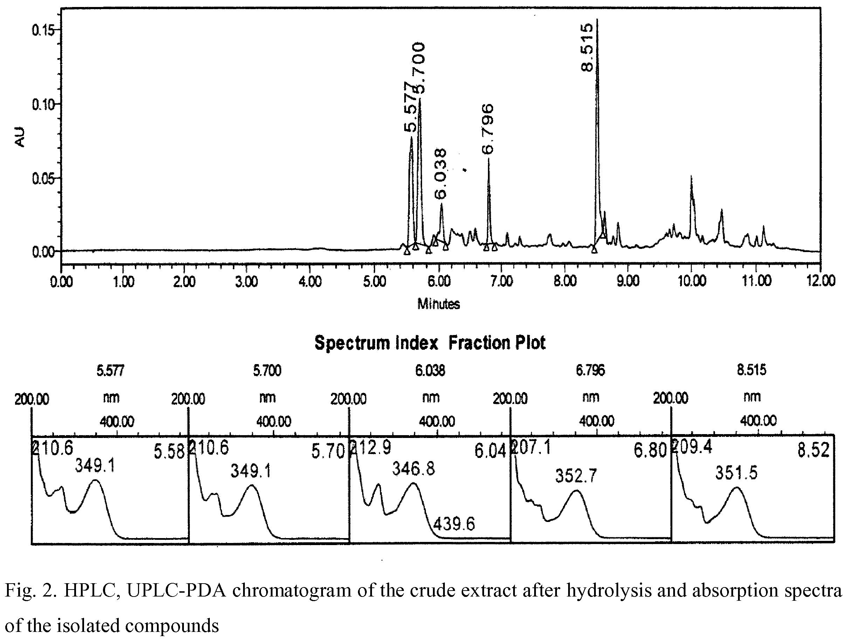

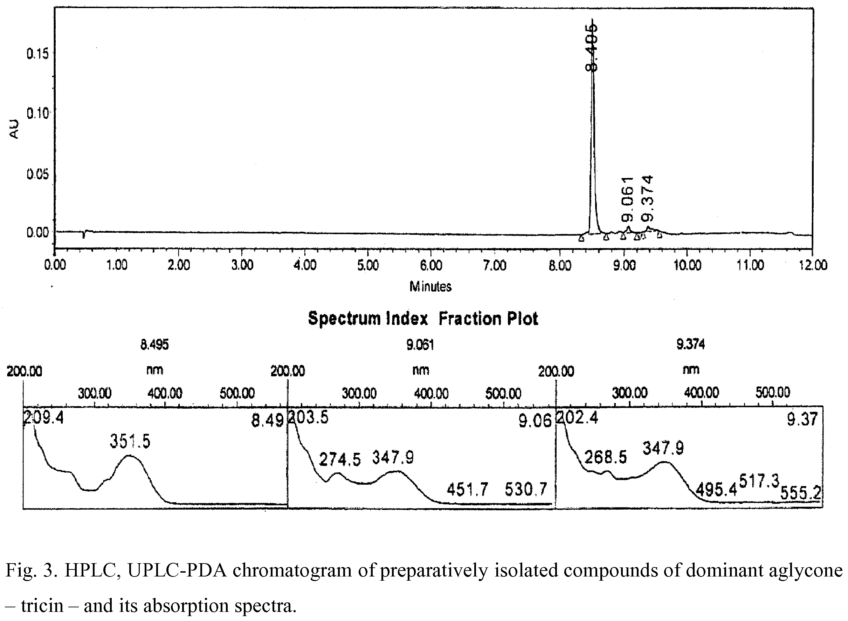

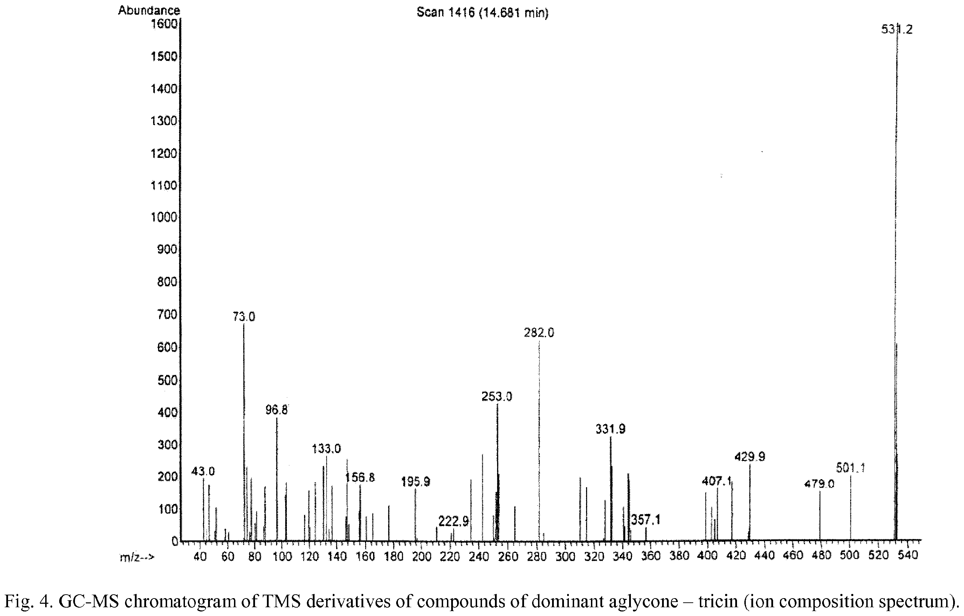

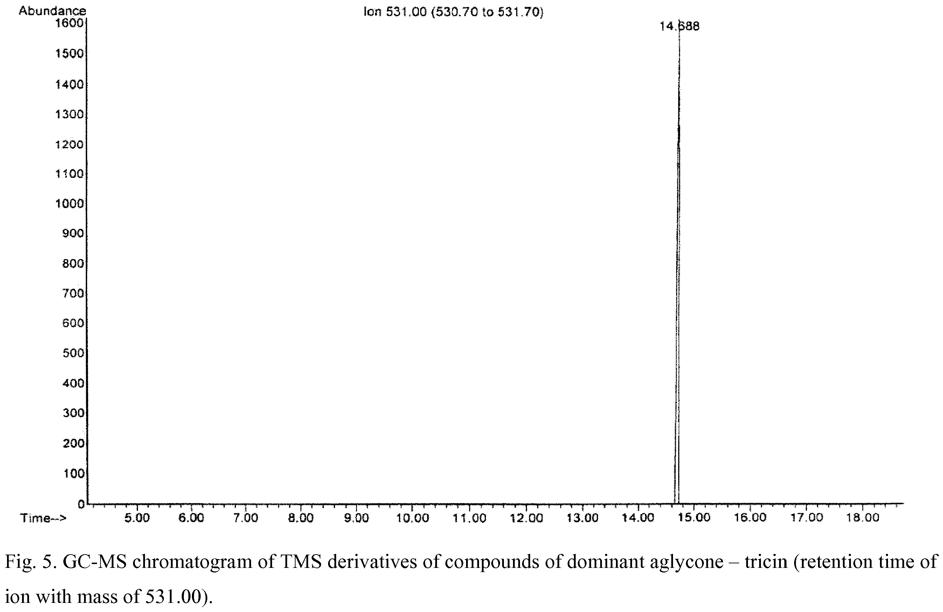

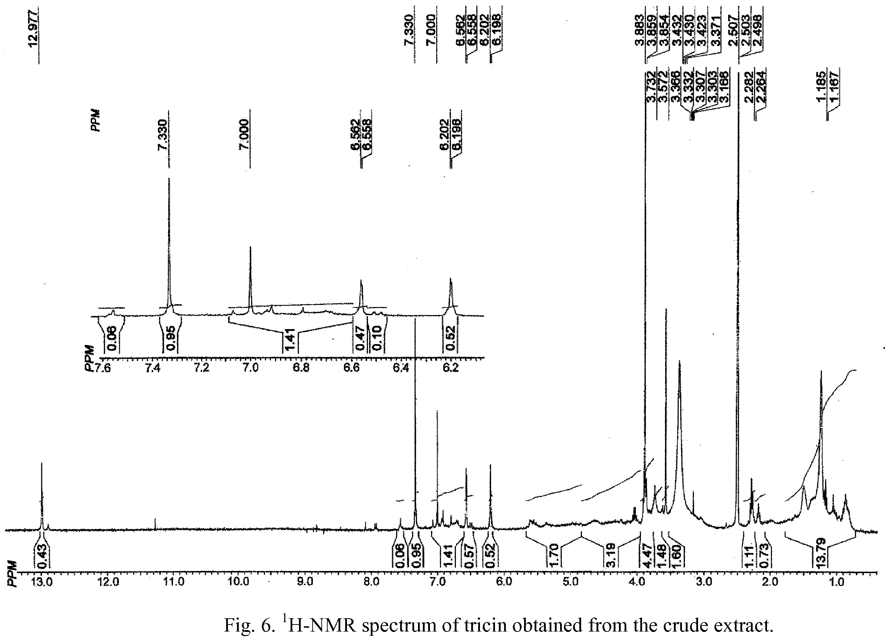

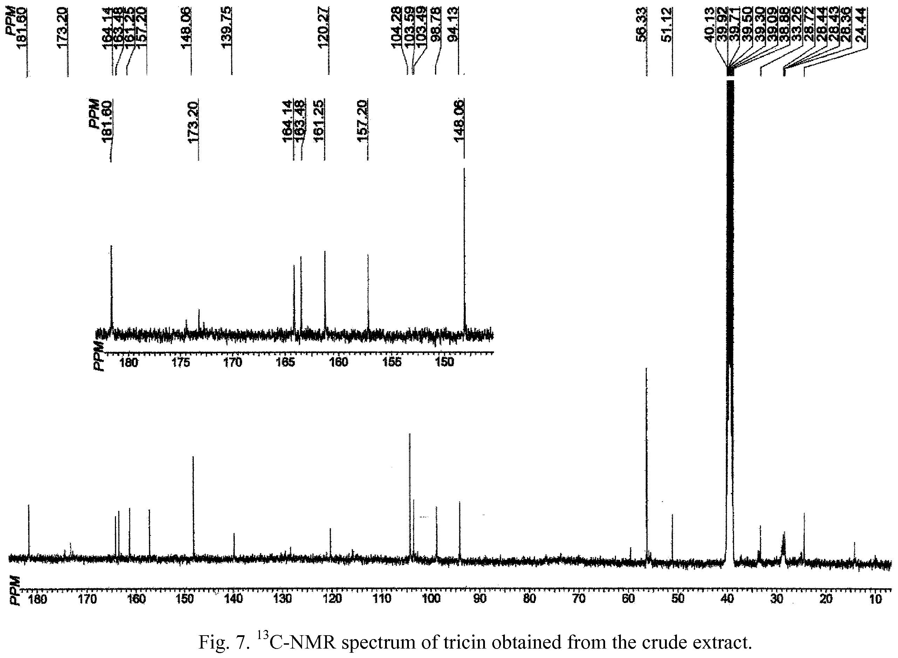

The chromatogram (Fig. 1) (HPLC, PDA detector) obtained before hydrolysis shows that there are three separate groups of compounds with different retention time: 5-7 min. (compounds of the group 1), 8-9.0 min. (compounds of the group 2) and compounds after the ninth minute (compounds of the group 3). The chromatogram obtained after hydrolysis (Fig. 2) shows that the compounds of the group 1 remained the same, while compounds of the group 2 and compounds of the group 3 increased in number. Compound of the group 2, which is dominant and has a typical spectrum for flavone, was isolated by preparative HPLC using Waters XBridge^{™} prep C₁₈ 10µm OBD™ 19 x 250 mm in a mode of reversed phase chromatography (mobile phase: methanol to water ratio is 1:9, flow rate is 15 ml/min.) with multiple injections and purification of the chromatographic fractions. As a result, 11 mg of aglycone were isolated (HPLC chromatogram is shown in Fig. 3). To determine the chemical formula and structure of the aglycone molecule, GC method was used with mass spectrometric detection. Since flavonoid compounds contain hydroxyl groups in their structure, which counteract their volatility, so to introduce the sample into the GC one must perform their derivatization. N, N-trimethylsilyl trifluoroacetamide (TMS derivatization) was used as a reagent. Chromatogram of aglycone derivative (Fig. 4, 5.) indicates that the mass of aglycone excluding the mass of fragments is 330. According to the catalogue of mass spectra this is tricin or rhamnazin. To clarify the structure of the molecule, NMR method on the proton and carbon nuclei was used. The following characteristics of the NMR spectra were obtained (Fig. 6, Fig. 7.): 13C (DMSO-d6, 100 MHz): δ = 56.33 (CH3 × 2), 94.13 (C-8), 98.78 (C-6), 103.49 (C-3), 103.59 (C-10), 104.28 (C-2'+ C-6"), 120.27 (C-1'), 139.75 (C-4'), 148.06 (C-3' + C-5'), 157.20 (C-5), 161.25 (C-7), 163.48 (C-9), 164.14 (C-2), 181.60 (C-4) of magnetic deviation.

1H (DMSO-d6, 400 MHz): δ = 3.88 (6H, c, CH3 × 2), 6.20 (1H, d, J = 2.0 Hz, H-6), 6.56 (1H, d, J = 2.0 Hz, H-8), 7.00 (1H, c, H-3), 7.33 (2H, c, H-2' + H-6 "), 12.98 (1H, c, OH-5) of magnetic deviation.

The signal at 12.98 of magnetic deviation is determined by the presence of the hydrogen bond between the hydrogen atom of the hydroxyl group and the oxygen atom of the carbonyl group. All other hydroxyl groups (OH-7 and OH-4') are not shown in the NMR spectrum because of their exchange with water.

Analysis of the NMR spectra data finally proves the authenticity of tricin as a main component of the compound 2. (Tricin or 5.7-Dihydroxy-2-(4-hydroxy-3,5 dimethoxyphenyl)-4*H*-chromen-4-one).

The detected structure of the dominant aglycone (tricin molecule) was confirmed by NMR using tricin standard (Hangzhou Dayangchem Co., LTD CAS. Na: 520-32-1 Purity: 98,5%), as well as by comparison of isolated tricin with tricin isolated from bamboo leaves according to the methods of Chinese researchers (J. Agric. Chem. 2007, 55, 10086-10092).

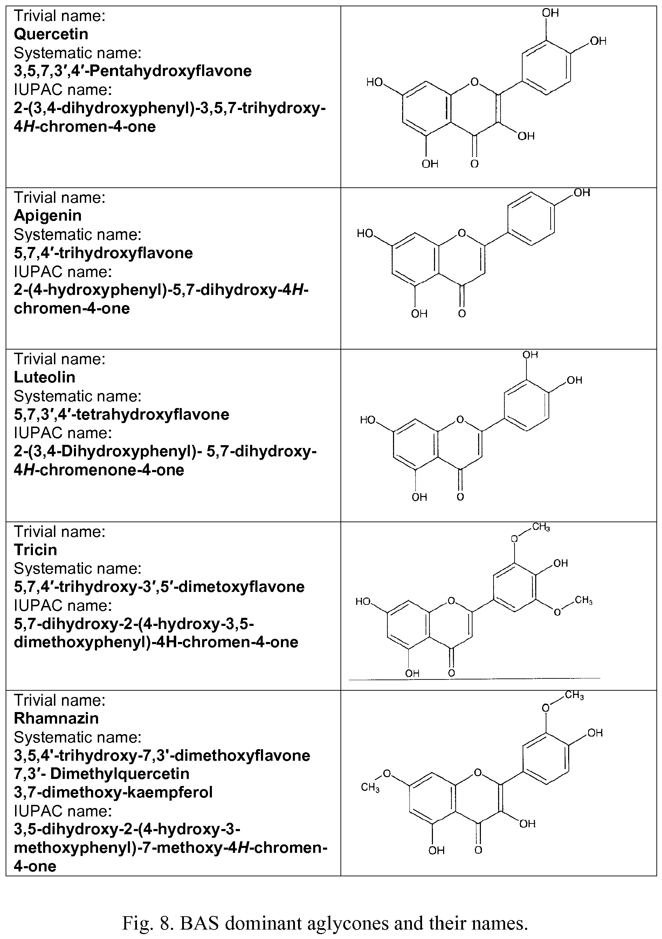

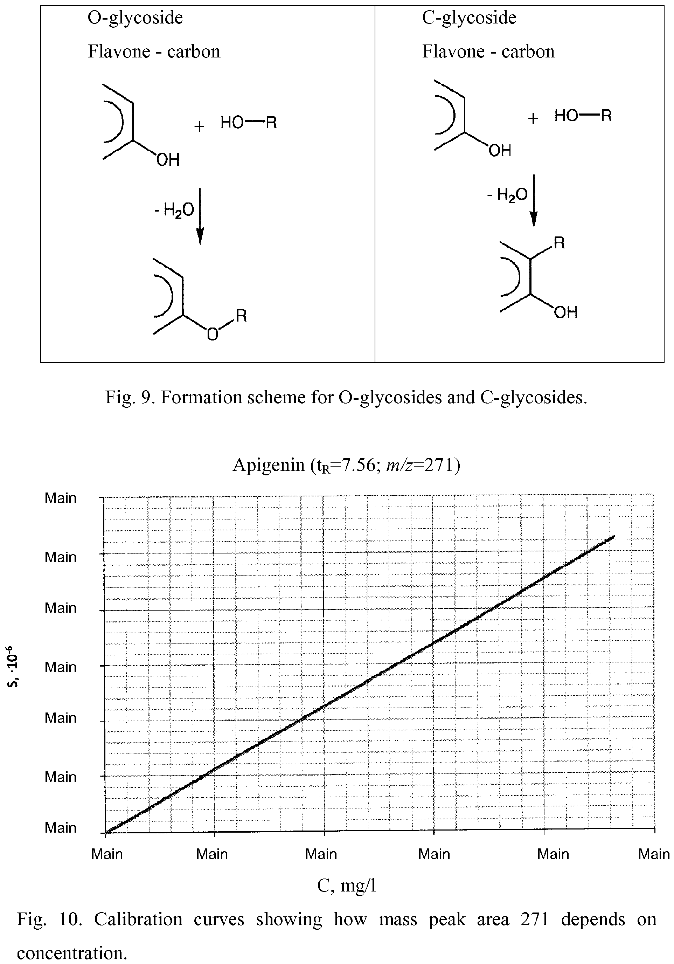

By the above procedure, presence of the flavonoid aglycones (except for tricin) was determined in the crude extract composition and their identification was conducted, namely: flavones - apigenin, luteolin; flavonols - quercetin, rhamnazin (Fig. 8). Flavonoid aglycones, known from the prior art (for example, U.S. Patent Application Nº. US 2008/0171708, A61K 31/702, dated June 17, 2006), and from our studies, are present in plant material and extracts prepared therefrom mostly in the form of O- and/or C-glycosides (Fig. 9), while individual aglycones may be simultaneously in the form of both O- and C-glycosides.

In order to determine separately the qualitative and quantitative composition of O- and C-glycosides one must first determine and identify the hydrocarbons present in the extract by GC with a mass-detector (using TMS derivatization) and UPLC with mass spectrophotometric detection and electrospray ionization (this method allows to determine the most intense fragment which corresponds to the molecular ion (m/z)). Hydrocarbon compounds in model extracts which have been identified (name, molecular weight) are listed in Table 1.

**Table 1.**

| **Identified hydrocarbon compounds in model extracts of Calamagrostis epigejos and Deschampsia cespitosa.** | | |
|---|---|---|
| **Item No.** | **Name** | **Molecular weight** |
| 1 | 2 | 3 |
| 1 | Glucose (Mannose) | 180 |
| 2 | 1.6-anhydro-β-α-glucose | 162 |
| 3 | Arabinose | 150 |
| 4 | Xylose | 150 |
| 5 | Ribose | 150 |
| 6 | Rhamnose | 164 |
| 7 | Glucofuranose | 180 |
| 8 | Glucuronic acid | 196 |
| 9 | D-sugar acid | 210 |
| 10 | Sorbose | 180 |
| 11 | 3-hydroxy propionic acid | 90 |
| 12 | 4-oxopentanoic acid | 118 |
| 13 | 1-Propene-1,2,3-tricarboxylic acid | 174 |
| 14 | 3-Hydroxy-butanoic acid | 104 |
| 15 | Butan | 72 |
| 16 | Succinic acid | 118 |
| 17 | Fumaric acid | 116 |
| 18 | Azelaic acid | 188 |
| 19 | 3-Methoxy-4-hydroxybenzoic acid | 168 |
| 20 | 3,4-dimethoxybenzoic acid | 182 |
| 21 | Benzoic acid | 122 |
| 22 | 4-Hydroxybenzoic acid | 138 |
| 23 | 3,4-dihydroxybenzoic acid | 154 |
| 24 | 2-carboxy-4-hydroxybenzoic acid | 182 |
| 25 | Syringic acid | 198 |
| 26 | Cinnamic acid | 118 |
| 27 | Caffeic acid | 180 |
| 28 | Ferulic acid (4-hydroxy-3-methoxycinnamic acid) | 194 |
| 29 | Sialic acid | 224 |
| 30 | Dimethoxycinnamic acid | 208 |
| 31 | alpha-Conidendrin | 356 |
| 32 | Aconitic acid | 174 |
| 33 | 3-(4-Hydroxy-3-methoxyphenyl) methyl ether of 2-propionic acid | 208 |

When the composition of hydrocarbones is determined, there is a need to determine conditions under which it is possible to distinguish between O- and C-glycosides, which empirical formulas are similar. In result of this, there is no difference between their optical characteristics and chromatographic fingerprinting. The most classical method is to determine the dynamics of aglycones release from glycocidic forms under destruction due to the effect of different concentrations of hydrogen ions. Acid hydrolysis is performed according to the method stated above using different concentrations of hydrochloric acid. Due to the remove of ethyl, the residue acid is dissolved in ethyl ether (9:1) and analyzed.

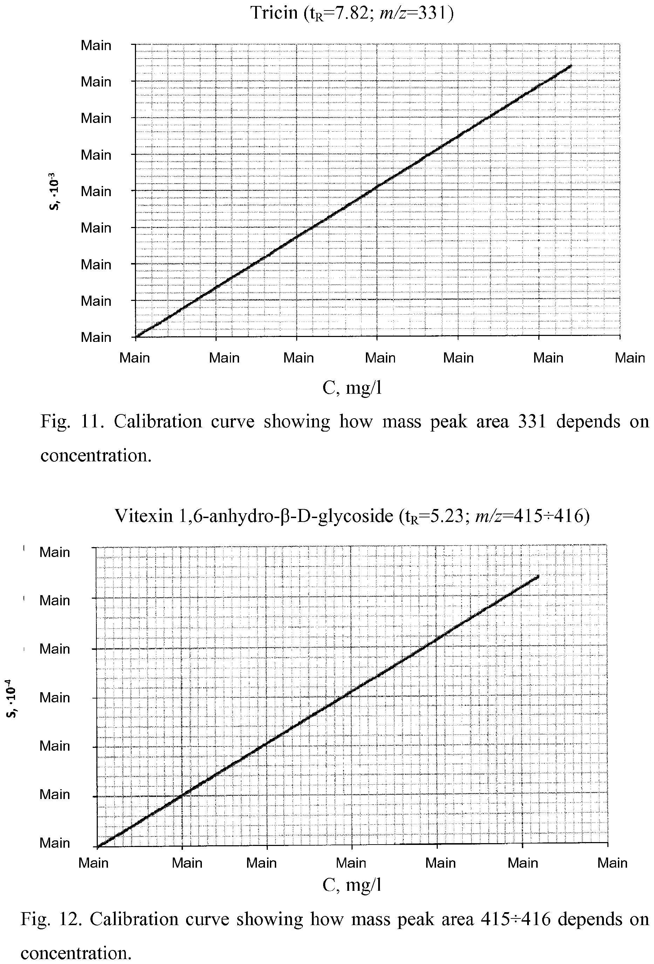

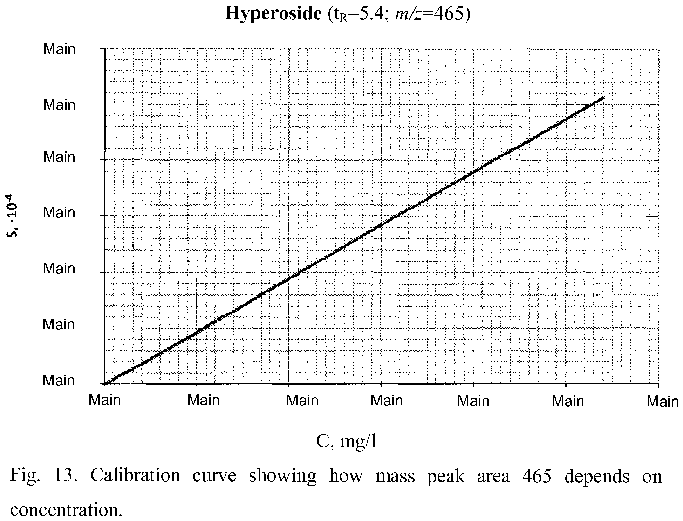

The hydrolysates obtained at different concentrations of hydrogen ions are analyzed by mass-spectrometry (UPLC-MS/MS). Quantitative determination of aglycones and/or glycosides according to the mass of individual ions is carried out using calibration curves with regard to standards: tricin, apigenin, luteolin, quercetin, rhamnazin, vitexin, hyperoside. Figures 10, 11, 12, 13 are examples of calibration curves for tricine, apigenin aglycones and hyperoside (O-glycoside) and vitexin 1,6-Anhydro-β-D-glycoside (C-glycoside).

Contents of aglycones in the extract and aglycones release dynamics of the glycosidic forms depending on the hydrogen ion concentration is shown in Table 2.

**Table 2**

| **Influence of hydrogen ion concentration on aglycones release** | | | | | | | |
|---|---|---|---|---|---|---|---|
| | Contents of aglycones, mg/l | | | | | | |
| Aglycone | Extract made of herbal mixture (1:1) | 0.08 mol HCl | | 1.2 mol HCl | | 1.6 mol HCl | |
| | | 1 | 2 | 1 | 2 | 1 | 2 |
| Luteolin | 1.5 | 2.0 | 2.0 | 2.1 | 1.9 | 2.0 | 2.0 |
| Tricin | 37.26 | 50.3 | 52.3 | 52.8 | 54.9 | 55.2 | 58.8 |
| Quercetin | 0.60 | 3.2 | 2.7 | 1.4 | 1.7 | 1.2 | 1.1 |
| Rhamnazin | 0.072 | 0.45 | 0.32 | 0.50 | 0.53 | 0.66 | 0.83 |
| Apigenin | 0.835 | 0.88 | 0.97 | 1.40 | 1.40 | 1.16 | 1.25 |

| | | | | | | | |
|---|---|---|---|---|---|---|---|
| 1 - when butylhydroxyanisole (BHA) is absent 2 - when BHA is present | | | | | | | |

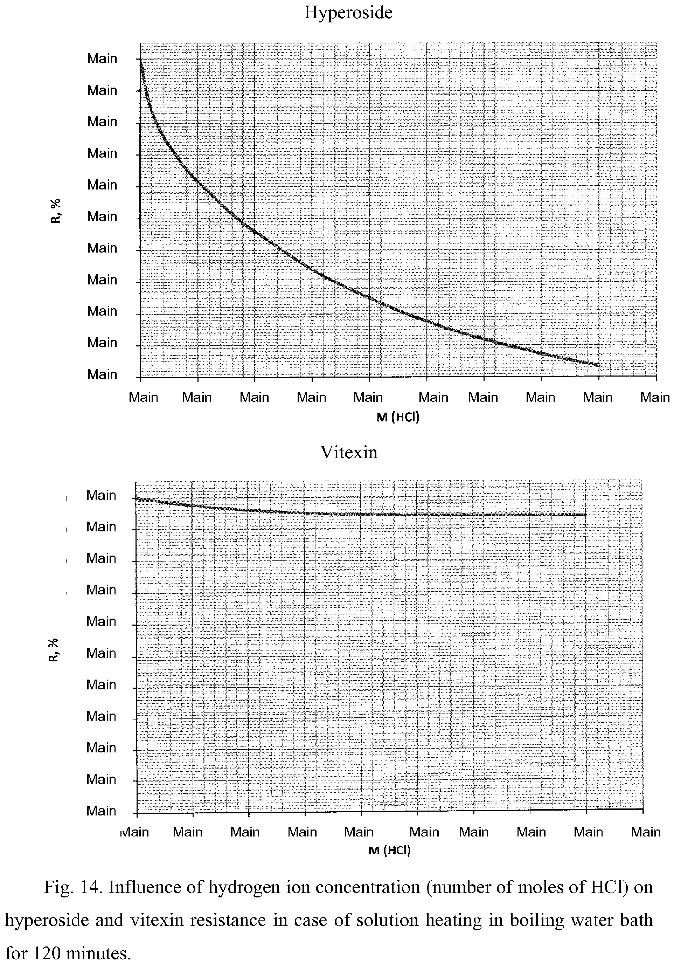

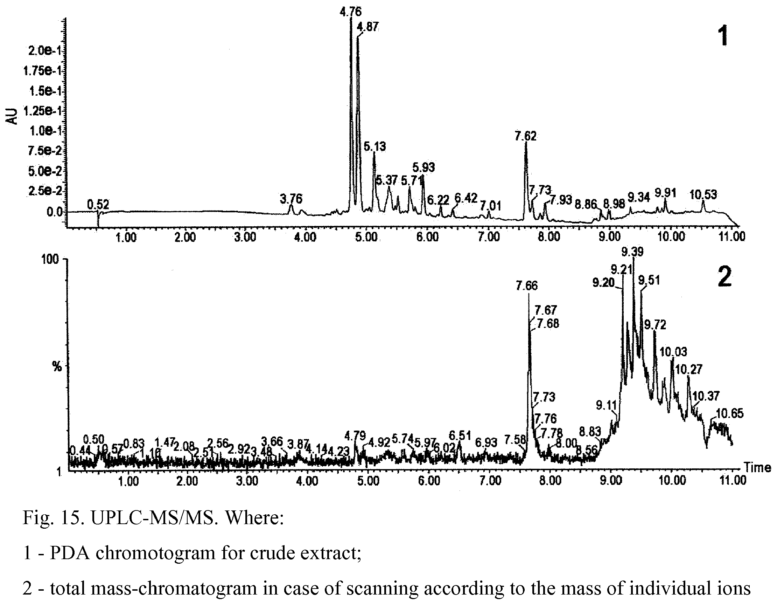

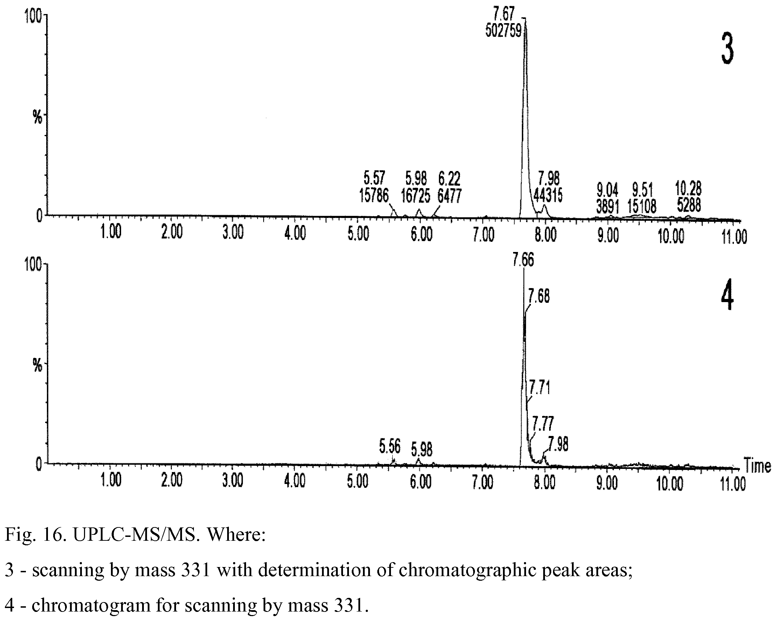

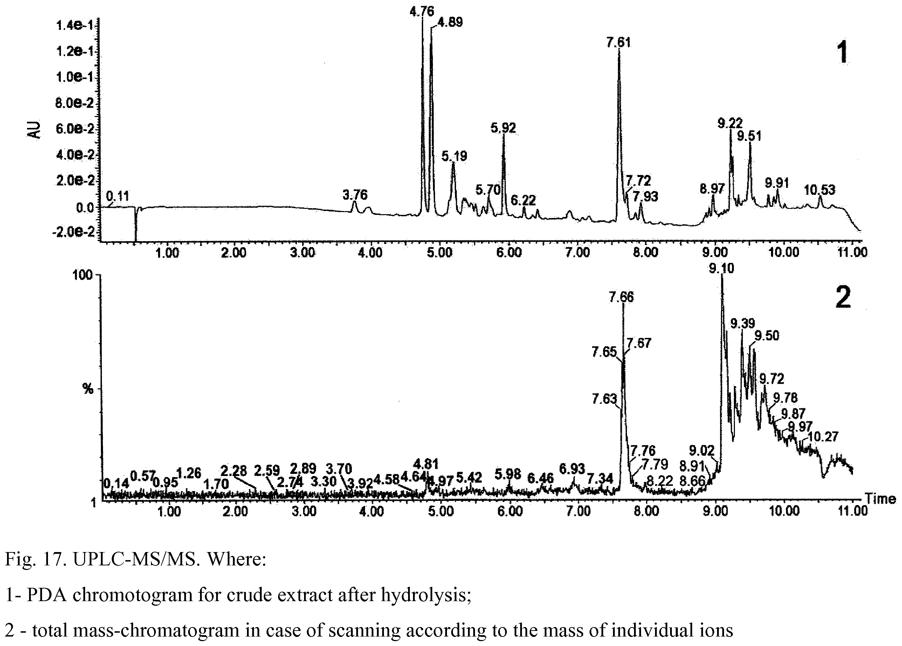

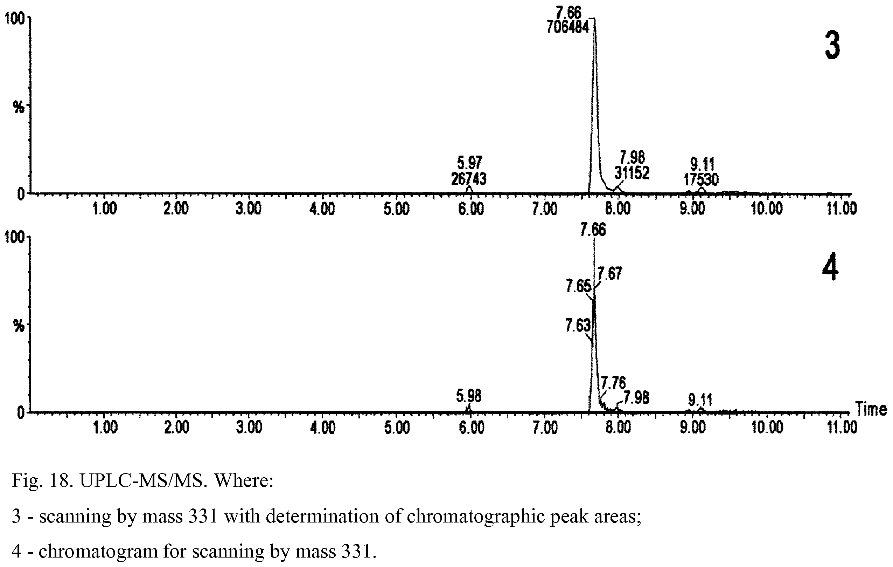

Most optimal conditions for quantitative determination of O- and C-glycosides are when the concentration of hydrogen ions is 0.08 Mol in relation to hydrochloric acid (HCl). Under such conditions O-glycosides are completely ruined, whereas C-glycosides remain (83%). (Dynamics of O- and C-glycosides ruining is shown by the example of vitexin and hyperoside in Fig. 14). Therefore, to obtain the information on quantitative content of aglycones, O- and C-glycoside forms of aglycones, one must analyze the results of extract and the product study after hydrolysis in HCl media of 0.08 Mol using such method as HPLC, UPLC-MS/MS. Fig. 15 shows an example of PDA chromatogram (1) the total mass chromatograms (2) with scanning of individual ions (UPLC-MS/MS retention time is slightly less than the retention time of Waters UPLS-PDA for the same column) of the crude extract before hydrolysis. In addition, Fig. 16 provides an example of scanning by mass 331 (tricin) with determination of chromatographic peak areas (3) and PDA chromatograms for scanning by mass 331 (4). Fig. 17 and Fig. 18 show an example of the crude extract scanning and scanning by mass 331 of crude extract after hydrolysis.

To calculate the amount of O-glycosides, the results of the analysis for the crude extract and the results obtained after hydrolysis were compared: absence or reduction of the peak after hydrolysis indicates the presence of O-glycoside, which may be present both independent (single chromatographic peak) and within the complex compounds (a decrease of chromatographic peak, which contains several compounds after hydrolysis). Theoretical values of the ion mass for glycoside compounds were used for calculations, taking into account the mass of the extracted aglycones, which are shown in Table 3, and identified carbohydrates (Table 1).

**Table 3**

| **Theoretically calculated glycosides for studied aglycones.** | | | | | | | | |
|---|---|---|---|---|---|---|---|---|
| Aglycone | Retention time (min) | Molar mass (M) | M +1 | Glucose (sorbose) | Xylose (ribose, arabinose) | 1,6-anhydro-β-D-glucose | Glucuronic acid | Rhamnose |
| Apigenin | 7.56 | 270 | 271 | 433 | 403 | 415 | 447 | 417 |
| Luteolin | 6.9 | 286 | 287 | 449 | 419 | 431 | 463 | 433 |
| Quercetin | 7.15 | 302 | 303 | 465 | 435 | 447 | 479 | 449 |
| Tricin (Rhamnazin) | 7.82 (9.02) | 330 | 331 | 493 | 463 | 475 | 507 | 477 |

In addition it was taken into account that in the case of O-glycoside (such as hyperoside) the detachment of aglycone molecule from hydrocarbon occurs as a result of electrospray application during mass spectrometry and the ratio between aglycone and initial glycoside is 100:1 In case of C-glycoside (such as vitexin) fragment OH + 1 , i.e. mass 18, is detached from the initial glycoside molecule and the ratio of peak areas of initial glycoside and its degradation product (also glycoside) is equal to 3.23. Studies identified the possible compounds which appear in the examined mass spectra and are formed by the degradation of C-glycosides (Table 4).

**Table 4**

| **Alteration products of flavonoid C-glycosides during mass spectrometry** | | | | |
|---|---|---|---|---|
| **Aglycone** | **Initial glycoside** | **M+1** | **Alteration product** | **M+1** |
| Tricin | tricin 8-C-glycoside | 493 | Anhydro C-glycoside | 475 |
| | C-glycoside with rhamnose | 477 | Anhydro C-glycoside | 459 |
| Rhamnazin | rhamnazin 8-C-xyloside | 463 | Anhydro C-glycoside | 448 |
| Apigenin | apigenin 8-C-glycoside | 433 | apigenin 8-C-(1,6-anhydro)-β-D-glycoside | 415 |
| | apigenin 8-C-xyloside | 403 | Anhydro C-glycoside | 385 |
| | C-glycoside with rhamnose | 417 | Anhydro C-glycoside | 399 |
| Luteolin | luteolin 8-C-glycoside | 449 | luteolin 8-C-(1,6-anhydro)-β-D-glycoside | 431 |
| | C-glycoside with xylose | 419 | Anhydro C-glycoside | 401 |
| | luteolin 8-C-glycoside | 433 | Anhydro C-glycoside | 415 |
| Quercetin | quercetin 8-C-glycoside | 465 | apigenin 8-C-glucuronide | 447 |
| | C-glycoside with xylose | 435 | Anhydro C-glycoside | 417 |
| | C-glycoside with rhamnose | 449 | Anhydro C-glycoside | 431 |

**Table 5**

| **Content of O-glycosides (mg/l) equivalent to hyperoside in the crude extract of herbal mixture (1:1) at a weight ratio of raw material to ethyl alcohol in the range from 1:10 to 3:1.** | | | | | |
|---|---|---|---|---|---|
| **m/z** | **Retention time, min** | **Weight ratio of raw material to ethyl alcohol (solid residue index %, density g/ml)** | | | **Compounds** |
| 1 | 2 | **1:10 (0.5; 0.8010)** | **1:2 (1.2; 0.8100)** | **3:1 (2.5; 0.8250)** | 4 |
| 449 | 4.9 | 11.23 | 84.20 | 140.50 | luteolin 7-O-glycoside |
| | 7.86 | 0.58 | 4.35 | 10.80 | quercetin 3-O-rhamnoside |
| 463 | 5.02 | 2.98 | 14.90 | 37.25 | rhamnazin 7-O-xyloside |
| | 5.86 | 1.52 | 7.80 | 19.50 | |
| 403 | 9.0 | 0.91 | 6.80 | 17.60 | apigenin 7-O-xyloside |
| 493 | 5.44 | 1.28 | 9.60 | 24.20 | tricin 7-O-glucoside |
| | 5.74 | 5.19 | 38.90 | 97.25 | |
| 431 | 4.9 | 17.70 | 125.00 | 210.20 | luteolin 7-O-(1,6-anhydro)-β-D-glycoside |
| 445 | 5.50 | 6.18 | 46.30 | 115.75 | tricin 7-O-(1,5-anhydro)-β-D-xyloside |
| | 5.03 | 18.00 | 135.00 | 247.50 | luteolin 7-O-anhydroglucuronide |
| 419 | 5.5 | 3.76 | 28.20 | 70.50 | luteolin 7-O-xyloside |
| 479 | 7.9 | 3.44 | 25.80 | 46.40 | quercetin 3-O-glucuronide |
| | 8.13 | 4.35 | 32.60 | 65.20 | |
| 465 | 4.94 | 1.66 | 12.40 | 31.00 | quercetin 3-O-glucoside |
| | 5.41 | 3.93 | 15.70 | 37.70 | quercetin 3-O-xyloside |
| | 8.92 | 0.07 | 0.30 | 0.75 | quercetin 7-O-rhamnoside |
| 582 | 4.91 | 15.24 | 114.30 | 244.30 | luteolin 3-O-glucoside, 7-O-rhamnoside |
| 639 | 5.74 | 7.24 | 54.30 | 119.45 | tricin 3-O-glucoside, 7-O-rhamnoside |
| 689 | 5.92 | 1.23 | 4.90 | 12.25 | quercetin 3-O-(4-hydroxybenzoyl) xyloside |
| | 6.26 | 8.16 | 40.80 | 102.00 | |
| | 6.45 | 5.27 | 26.10 | 65.25 | |
| | 7.03 | 2.85 | 11.40 | 28.50 | |
| | Total of O-glycosides (mg/l) | 122.75 | 842.95 | 1726.25 | |

**Table 6**

| **Content of C-glycosides (mg/l) equivalent to hyperoside in the crude extract of herbal mixture (1:1) at a weight ratio of raw material to ethyl alcohol in the range from 1:10 to 3:1** | | | | | |
|---|---|---|---|---|---|
| **m/z** | **Retention time, min.** | **Weight ratio of raw material to ethyl alcohol (solid residual index %, density g/ml)** | | | **Compounds** |
| 1 | 2 | **1:10 (0.5; 0.8010)** | **1:2 (1.2; 0.8100)** | **3:1 (2.5; 0.8250)** | 4 |
| 465 | 4.94 | 0.36 | 2.02 | 4.95 | quercetin 8-C-glucoside |
| | 5.29 | 0.28 | 2.23 | 5.58 | |
| | 5.51 | 0.12 | 1.00 | 2.48 | |
| | 5.51 | 0.11 | 1.06 | 2.43 | |
| | 9.12 | 1.24 | 8.60 | 19.78 | |
| | 9.12 | 1.89 | 11.30 | 27.69 | |
| 403 | 9.0 | 10.26 | 66.75 | 166.87 | apigenin 8-C-glycoside |
| | 9.0 | 0.32 | 1.83 | 4.09 | |
| 463 | 5.02 | 1.15 | 6.90 | 17.25 | tricin 8-C-xyloside |
| | 5.35 | 1.20 | 9.00 | 24.30 | |
| | 5.47 | 1.80 | 11.70 | 28.08 | |
| | 5.47 | 0.02 | 0.09 | 0.22 | luteolin 8-C-glucuronide |
| | 5.57 | 0.98 | 6.86 | 16.81 | |
| | 9.15 | 1.65 | 10.00 | 23.70 | |
| 493 | 5.75 | 1.08 | 7.00 | 17.50 | tricin 8-C-glucoside |
| | 5.97 | 2.12 | 15.90 | 36.57 | |
| | 5.97 | 1.54 | 10.00 | 23.17 | |
| | 9.16 | 0.68 | 4.15 | 10.17 | rhamnazin 8-C-glucoside |
| | 9.11 | 4.94 | 34.60 | 89.96 | |
| 507 | 8.95 | 0.11 | 0.65 | 1.69 | tricin 8-C-glucuronide |
| | 9.08 | 1.44 | 10.0 | 24.51 | |
| 415 | 9.16 | 1.78 | 10.50 | 25.73 | apigenin 8-C-(1.6-anhydro)-β-D-glucoside |
| 447 | 5.47 | 0.38 | 2.30 | 5.64 | apigenin 8-C-glucuronide |
| | 5.65 | 2.02 | 13.10 | 31.40 | |
| | 7.89 | 0.64 | 3.20 | 7.36 | |
| 433 | 4.86 | 0.31 | 1.85 | 4.45 | apigenin 8-C-glucoside |
| | 4.86 | 0.24 | 1.08 | 2.82 | |
| | 5.33 | 1.98 | 12.70 | 31.12 | |
| | 5.33 | 3.04 | 18.40 | 44.20 | |
| | 9.06 | 3.85 | 27.20 | 62.56 | |
| | 9.06 | 1.56 | 8.40 | 17.82 | |
| 479 | 7.22 | 0.12 | 0.70 | 1.75 | quercetin 8-C-glucuronide |
| | 7.89 | 0.97 | 6.80 | 15.23 | |
| | 8.09 | 0.85 | 6.15 | 15.98 | |
| | 9.0 | 0.72 | 4.40 | 9.94 | |
| | 9.14 | 0.73 | 4.60 | 8.75 | |
| 449 | 4.9 | 3.12 | 20.30 | 56.35 | luteolin 8-C-glucoside |
| | 5.02 | 4.95 | 37.10 | 89.04 | |
| | 5.02 | 3.12 | 20.30 | 49.74 | |
| | 7.86 | 1.54 not | 10.70 | 23.84 | |
| | 8.01 | defined | 0.041 | 0.09 | |
| | 8.06 | 2.46 | 16.05 | 40.43 | |
| | 9.12 | 2.60 | 14.50 | 35.67 | |
| 431 | 4.9 | 8.04 | 56.30 | 129.50 | luteolin 7-O-(1,6-anhydro)-β-D-glucoside |
| | 5.33 | 4.27 | 25.60 | 62.72 | |
| | 9.06 | 4.5 | 27.80 | 63.94 | |
| 671 | 5.62 | 0.24 | 1.60 | 4.00 | quercetin 8-C-(sinapoil)-glucoside |
| | 9.08 | 0.63 | 4.10 | 9.27 | |
| Total of C-glycosides (mg/l) | | 87,65 | 577.36 | 1395.71 | |

Thus, as illustrated by the analysis of the crude extract, qualitative and quantitative determination of the free aglycones (Table 2), as well as the glycosidic forms of aglycones was performed, namely: O-glycosides (Table 5); C-glycosides (Table 6).

On the HPLC chromatogram, the compounds of group 1 (Fig. 1), with the lowest retention time as the most hydrophilic, are a mixture of O- and C-glycosides of aglycones isolated above, within which tricin, luteolin, apigenin are predominant. The compounds of group 2 (Fig. 1) are a mixture of free aglycones. The compounds of group 3 are acylated forms of O-, C-, and bi-glycosides.

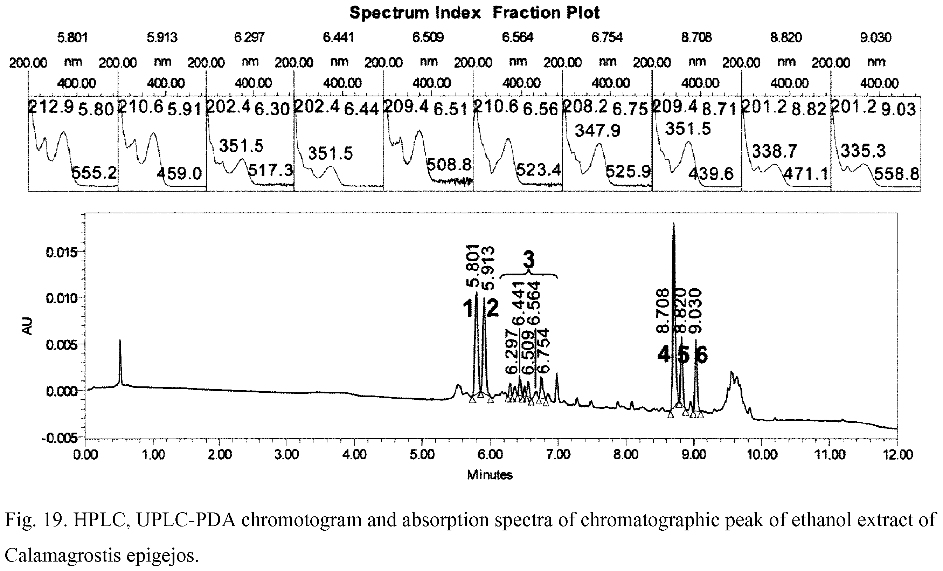

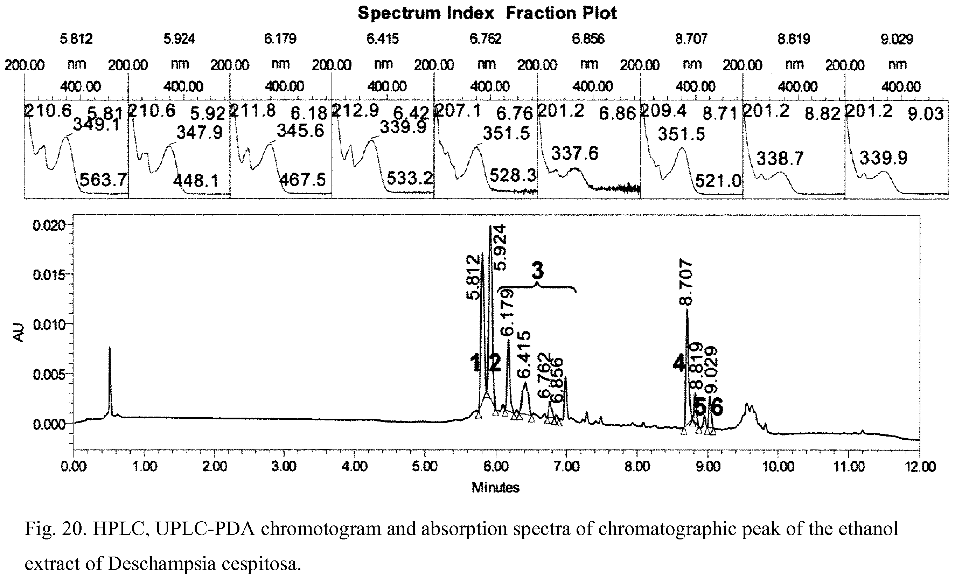

The above results of the determination of qualitative and quantitative composition of aglycones and their glycosides in the extract made from a mixture of herbs for which the biological activity was studied, allow to compare the composition and the biological activity of extracts of each herb separately. To determine differences in the composition and for comparative studies of specific biological effects, model extracts made of each herb separately were produced with a calibrated amount of flavonoids and their glycosides, equal to 0.22 mg/ml, at a weight ratio of raw material to ethyl alcohol equal to 1:4.5 and with the solid residue index equal to 0.7% (the average value of density is 0.8080 g/ml). Typical HPLC chromatograms (detection at 350 nm) under the conditions of chromatography given hereinabove and absorption spectra of compounds of ethanol extracts of herbs Calamagrostis epigejos and Deschampsia cespitosa are shown in Fig. 19 and Fig. 20, respectively.

These chromatograms show that the qualitative composition of extracts is the same. Differences in composition of ethanolic extracts are explained by different quantitative content of individual molecules of flavonoids and their O- and C-glycosides.

**Table 7**

| **Comparative composition and deviations (mg/l) of free aglycones, O and C-glycosides content in model extracts of herb mixture (1:1) and of each herb separately at a weight ratio of raw material to ethyl alcohol in the range of 1:10 to 3:1.** | | | | | | | | | | |
|---|---|---|---|---|---|---|---|---|---|---|
| **No.** | **Compounds** | **Extract made of herb mixture (1:1)** | | | **Extract made of Calamagrostis epigejos** | | | **Extract made of Deschampsia cespitosa** | | |
| | | **Weight ratio of raw material to ethyl alcohol (solid residual index %, density (g/ml))** | | | | | | | | |
| | | **1:10** | **1:2** | **3:1** | **1:10** | **1:2** | **3:1** | **1:10** | **1:2** | **3:1** |
| | | **0.5;0.8 010** | **1.2;0. 8100** | **2.5;0.82 50** | **0.5;0.80 10** | **1.2;0.81 00** | **2.5;0.82 50** | **0.5;0.80 10** | **1.2;0.81 00** | **2.5;0.82 50** |
| 1 | Tricin | 4.000 | 37.260 | 95.000 | 0.900 | 9.450 | 24.00 | 0.600 | 6.800 | 14.000 |
| 2 | Luteolin | 0.100 | 1.500 | 3.600 | 0.004 | 0.040 | 0.010 | 0.100 | 1.250 | 3.200 |
| 3 | Apigenin | 0.080 | 0.840 | 1.800 | 0.020 | 0.200 | 0.600 | 0.010 | 0.150 | 0.500 |
| 4 | Quercetin | 0.060 | 0.600 | 1.400 | 0.030 | 0.300 | 0.800 | 0.010 | 0.100 | 0.500 |
| 5 | Rhamnazin | 0.010 | 0.070 | 0.140 | 0.010 | 0.020 | 0.080 | 0.010 | 0.020 | 0.080 |
| 6 | Total, free aglycones | 4.250 | 40.270 | 101.940 | 0.964 | 10.010 | 25.490 | 0.730 | 8.320 | 18.280 |
| 7 | tricin O-glycosides | 19.92 | 149.10 | 356.65 | 1.00 | 10.0 | 21.00 | 1.00 | 10.5 | 21.00 |
| 8 | tricin C-glycosides | 10.44 | 71.15 | 173.07 | 0.20 | 6.2 | 11.00 | 1.00 | 10.0 | 22.00 |
| 9 | apigenin O-glycosides | 0.91 | 6.80 | 17.60 | 0.60 | 8.9 | 22.00 | 1.00 | 11.35 | 24.00 |
| 10 | apigenin C-glycosides | 26.38 | 167.30 | 402.63 | 0.05 | 0.53 | 1.20 | 0.04 | 3.70 | 9.00 |
| 11 | luteolin O-glycosides | 65.93 | 486.70 | 913.00 | 0.01 | 2.36 | 6.80 | 4.00 | 41.6 | 120.0 |
| 12 | luteolin C-glycosides | 36.95 | 245.60 | 592.05 | 0.02 | 0.22 | 0.06 | 0.50 | 6.95 | 21.00 |
| 13 | quercetin O-glycosides | 31.49 | 177.65 | 399.85 | 0.01 | 0.16 | 0.04 | 0.02 | 0.18 | 0.40 |
| 14 | quercetin C-glycosides | 8.26 | 54.56 | 127.83 | 0.02 | 0.22 | 0.70 | 0.02 | 0.18 | 0.70 |
| 15 | rhamnazin O-glycosides | 4.50 | 22.70 | 56.75 | 6.50 | 67.5 | 180.00 | 10.00 | 108.0 | 260.00 |
| 16 | rhamnazin C-glycosides | 5.62 | 38.75 | 100.13 | 0.01 | 0.08 | 0.12 | 0.01 | 0.12 | 0.36 |
| 17 | Total of O-glycosides | 122.75 | 842.95 | 1726.25 | 8.31 | 70.02 | 227.84 | 16.02 | 171.63 | 454.40 |
| 18 | Total of C-glycosides | 87.65 | 577.36 | 1395.71 | 0.60 | 7.25 | 13.08 | 2.57 | 20.95 | 53.06 |

With help of techniques that were used to determine the crude extract by HPLC-MS/MS one can determine the qualitative and quantitative composition of extracts for each herb separately.

As shown in Table 7, the content of free aglycones in extracts of Calamagrostis epigejos ranges from 9.5% to 11.5%, whereas for extracts of Deschampsia cespitosa it ranges from 3.8% to 4.2% of the total amount of flavonoid compounds and it is virtually independent of ratio of raw materials to the extractant in the specified range. These data indicate the constancy of the composition of extractives in model extracts.

The highest percentage (concentration) of the mixture of flavonoid compounds (aglycones and their O- and/or C-glycosides) is present in the extract of herb mixture (1:1) at a weight ratio of raw materials to the extractant equal to 3:1, equal to 9.408% (3223.9 mg/l) of the solid residue value: 30300 mg/l. The smallest amount of flavonoid compounds (0.132% or 9.870 mg/l with solid residue value equal to 6240 mg/l) is present in the extract of Calamagrostis epigejos at a ratio of raw materials to the extractant equal to 1:10.

When the ratio of raw materials to the extractant is less than 1:10, flavonoid compounds of luteolin, apigenin and quercetin became non-extractable and the material loses its antiviral properties. By increasing the ratio of raw materials to the extractant of 3:1 the amount of extractable O- and/or C-glycosides is not increased, extracts lose antiviral properties because they are unstable in time due to the deposition of the coagulative sediment.

Comparative tests of biological activity were carried out in parallel and simultaneously for the crude extract of herb mixture (1:1) and individual extracts obtained from Calamagrostis epigejos and Deschampsia cespitosa with calibrated content of flavonoid amount of 0.22 mg/ml.

These examples illustrate the properties of biologically active substances, but do not limit the patent. The observed properties are shown in Tables No.1-16 and Figures 1-27.

The terms used in the application and their abridged versions, research techniques are formally adopted (Doklinicheskie issledovaniya lekarstvennykh sredstv (metodicheskie rekomendatsyi) [Preclinical trials of medicinal products (guidelines)]. Edited by Corresponding Member of Academy of Medical Sciences of Ukraine O.V. Stefanova - Kyiv: Avicenna, 2001 - 528 p. (in Russian)).

### INVENTION EMBODIMENTS

### Example 1.

Study of anti-herpetic activity of model extracts in *in vitro* experiments.

To evaluate anti-herpetic activity of extracts we used passaged culture of Vero cells (renal cells from monkey). The cells were cultivated in plates with the medium of RPMI-1640 +10% fetal serum (Nunclon, Surface, Denmark) at temperature 37°C in thermostat with CO₂ feed.

The type 2 herpes virus, "VN" strain (HSV) was used, infectious titer: 5.0÷7.0 1g TCD₅₀ /0.1 ml (TCD₅₀ - tissue cytopathic dose of the virus that causes damage in 50% of cell monolayer). For examination of antiviral activity the one-day cultures of Vero cells with confluent cell monolayer were used. Growth medium was removed and the different concentrations of extracts were applied on the cell monolayer. In one hour after the contact, the herpes virus in dose of 100 TCD₅₀ was added, and the supporting medium was placed into the wells (serum-free nutritive medium).

Cultures were incubated in thermostat with CO₂ feed during 5 days, with daily microscopic control and registration of viral reproduction by a HSV cytopathic effect on Vero cells in comparison with control cultures, where the monolayer was not treated with extracts (control of virus).

A HSV cytopathic effect on cells is morphologically expressed as formation of symplasts or round cells along with proliferation and giant multinuclear cells.

After 3 days, cultural medium was collected from the plate wells and the infectious titer was determined in each probe after application of different doses of model extracts. Results of HSV-2 reproduction are provided in the Table 8.

**Table 8**

| **Results of the effect of the experimental extracts on HSV-2 reproduction in Vero cells culture and determination of minimal active concentration (MAC)** | | | |
|---|---|---|---|
| Concentration of flavonoids in diluted extracts, mcg/ml | Names of extracts and their effect on infectious titers in 1g ID₅₀ | | |
| | Crude extract (1:1) | Extract of Calamagrostis epigejos | Extract of Deschampsia cespitosa |
| 0.272 | 3.0 | 3.0 | 3.0 |
| 0.136 | 4.0 | 5.0 | 4.0 |
| 0.068 | 5.0 | 4.0 | 4.0 |
| 0.034 | 5.0 | 5.0 | 3.0 |
| 0.017 | 6.0 | 6.0 | 4.0 |
| Control of virus | 7.0 | 7.0 | 7.0 |

Table 9 provides parameters of Maximal Tolerable Concentration (MTC), Minimal Active Concentration (MAC), and Chemotherapeutic Index (CTI).

**Table 9.**

| **Results of determination of MTC, MAC, and CTI of model extracts during their effect on the type 2 herpes virus in Vero cells culture.** | | | |
|---|---|---|---|
| Parameters | Values of MTC, MAC, and CTI | | |
| | Crude extract (1:1) | Extract of Calamagrostis epigejos | Extract of Deschampsia cespitosa |
| MTC mcg/ml | 5.5 | 5.5 | 2.7 |
| MAC mcg/ml | 0.034 | 0.034 | 0.017 |
| CTI | 161.7 | 161.7 | 158.8 |

The examined extracts effectively inhibit the type 2 herpes virus. Minimal active concentration that causes inhibition of a virus specific effect by 2.0-3.0 1g TCD₅₀ was 0.017 mcg/ml for extract of Deschampsia cespitosa, which is twice less than the value of minimal active concentration (0.034 mcg/ml) for the crude extract and the extract of Calamagrostis epigejos. CTI of all these extracts was approximately 160.

### Example 2.

Determination of anti-influenza activity in *in vitro* experiments.

The influenza virus of A/FM, 1/47 (H1N1) strain, infectious titer of allantoic culture - 6.0 1g EID₅₀/0.2 ml, was used.

For *in vitro* determination of antiviral activity of extracts, one day passaged culture of MDCK cells (renal cells from dog) with confluent layer was used. Cells were cultivated in plates on medium of RPMI-1640+10% fetal serum (Nunclon, Surface, Denmark) at temperature 37°C in thermostat with CO₂ feed. For enhance of cell sensitivity to contamination with the influenza virus, the processing with trypsin was performed. Trypsin stock solution was prepared by addition of up to 3 g of weighted amount of enzyme into 3 ml of DMEM medium. Cells were washed with this solution for three times - 50 ml into each well.

Growth medium was removed, different concentrations of model extracts were added into cells and the influenza virus was applied in dose of 100 TCD₅₀.

Cultures were incubated in thermostat with CO₂ feed during 3 days, with daily microscopic control.

After 72 hours of incubation of cells, cultural fluid was collected and the infectious titer of the influenza virus was determined in it by titrating in cell culture. Table 10 provides results of determination of an effect of model extracts on the influenza virus. According to obtained data, model extracts in different concentrations inhibit reproduction of the influenza virus by 2.0-3.0 1g ID₅₀, which confirms the anti-influenza effect of the compounds in the crude extract as well as in each herb separately.

**Table 10**

| **Results of the effect of experimental extracts on influenza virus reproduction in MDCK cell culture and determination of minimal active concentration (MAC)** | | | |
|---|---|---|---|
| Concentration of flavonoids in diluted extracts, mcg/ml | Names of extracts and their effect on infectious titers in 1g ID₅₀ | | |
| | Crude extract (1:1) | Extract of Calamagrostis epigejos | Extract of Deschampsia cespitosa |
| 0.055 | 3.0 | 4.0 | 3.0 |
| 0.0275 | 2.0 | 4.0 | 3.0 |
| 0.0137 | 3.0 | 7.0 | 3.0 |
| 0.0068 | 3.0 | 6.0 | 2.0 |
| 0.0034 | 5.0 | 6.0 | 3.0 |
| 0.0017 | 5.0 | 6.0 | 7.0 |
| Control of virus | 6.0 | 6.0 | 6.0 |

Chemotherapeutic index (CTI) of model extracts for the influenza virus was determined by evaluation of correlation of maximal tolerated concentration (MTC) in minimal active concentration (MAC), in which the virus-specific cytopathic action is inhibited by 2.0-3.0 1g ID₅₀ (Table 11).

**Table 11**

| **Results of determination of MTC, MAC and CIT of model extracts during their action on the influenza virus in MDCK cell culture.** | | | |
|---|---|---|---|
| Parameters | Values of MTC, MAC and CTI | | |
| | Crude extract (1:1) | Extract of Calamagrostis epigejos | Extract of Deschampsia cespitosa |
| MTC mcg/ml | 5.5 | 5.5 | 1.8 |
| MAC mcg/ml | 0.0068 | 0.0275 | 0.0034 |
| CTI | 808.8 | 200.0 | 52.9 |

All model extracts are effective against the influenza virus. The greatest decrease of the infectious titer of the influenza virus (by 3.0 1g ID₅₀) is observed for BAS concentration equal to 0.0034 mcg/ml, for the effect of extract of Deschampsia cespitosa. The maximal selectivity of the effect on the influenza virus is shown by the crude extract, where CTI = 808.

### Example 3.

Investigation of activity of extracts against the hepatitis C virus (HCV).

The bovine viral diarrhea virus (BVDV) was chosen as the surrogate HCV virus as it is the test-model of the hepatitis C virus.

Antiviral activity was investigated in MDBK culture, which was processed with the use of different dilutions of extracts and where BVDV in dose of 100 TCD₅₀ was added. Cultures were incubated in thermostat till appearance of the specific cytopathic effect of the virus in control, and then the infectious titer of the virus was determined in the cultural medium.

Results are provided in table 12.

**Table 12**

| **Results of the effect of experimental extracts on reproduction of the bovine viral diarrhea virus and determination of minimal active concentration (MAC) in MDBK cell culture.** | | | |
|---|---|---|---|
| Concentration of flavonoids in diluted extracts, mcg/ml | Names of extracts and their effect on the infectious titer of the surrogate HCV virus in 1g ID₅₀ | | |
| | Crude extract (1:1) | Extract of Deschampsia cespitosa | Extract of Calamagrostis epigejos |
| 0.275 | 4.0 | 4.0 | 4.0 |
| 0.137 | 4.0 | 4.0 | 4.0 |
| 0.068 | 3.0 | 4.0 | 4.0 |
| 0.034 | 4.0 | 3.0 | 4.0 |
| Control of virus | 8.0 | 8.0 | 8.0 |

The analysis of obtained results shows that extracts inhibit reproduction of the surrogate hepatitis C virus by 3-2 1g ID₅₀.

Results of determination of MTC, MAC, CTI for effect of the crude extract, extracts of Deschampsia cespitosa, Calamagrostis epigejos on the BVDV virus in MDBK cell culture are provided in table 13.

**Table 13**

| **Results of determination of MTC, MAC and CIT of model extracts during their action on BVDV in MDBK cell culture.** | | | |
|---|---|---|---|
| Parameters | Values of MTC, MAC and CTI for model extracts | | |
| | Crude extract (1:1) | Extract of Deschampsia cespitosa | Extract of Calamagrostis epigejos |
| MTC | 5.5 | 2.7 | 5.5 |
| MAC | 0.034 | 0.034 | 0.034 |
| CTI | 161.7 | 79.4 | 161.7 |

Results of investigation showed that model extracts are effective inhibitors of the surrogate hepatitis C (VBDV) virus with high value of CTI.

All extracts inhibit the bovine diarrhea virus (test-model of hepatitis C virus) by 4.0-5.0 1g ID₅₀ in concentrations equal to 0.0034 mcg/ml.

### Example 4.

Interferon inducing activity of model extracts *in vivo.*

Interferon inducing activity was examined in *in vivo* experiments in white outbred mice, that underwent intraperitoneal administration of extracts (0.1 ml) in concentration of 55.5 mcg/kg. After 24 hours mice were removed from experiment through euthanasia, and interferon (IFN) was determined in their blood serum with commonly used method of inhibition of cytopathic action (CPA) of the vesicular stomatitis virus (VSV) in homological tissue culture L929. The type of interferon was determined by an acidic sensitivity marker. For this purpose, blood serum was divided into two same parts. In one of them pH of liquid was made up to 2.0, using 4 N HC1, and left for 24 hours in 4 °C, then pH of liquid was restored up to 7.2 using 4 N NaOH.

Results of performed experiment are provided in table 14.

**Table 14**

| Interferon levels in blood serum of mice that underwent administration of model extracts. | | |
|---|---|---|
| Model extracts and reference inducer | Units of interferon activity per ml | |
| | -pH | +pH |
| Crude extract | 320 | 0 |
| Extract of Deschampsia cespitosa | 320 | 0 |
| Extract of Calamagrostis epigejos | 1280 | 0 |
| Poly I: Poly C | 1280 | 1280 |

| | | |
|---|---|---|
| Poly I: Poly C is a reference inducer of IFN (Calbiocheus, USA) | | |

The marker of pH sensitivity allowed to determine that all three extracts induce γ-interferon.

All extracts have the ability to induce the immune γ-interferon. The extract of Calamagrostis epigejos induces interferon at the level of a reference inducer Poly I: Poly C, and the crude extract and the extract of Deschampsia cespitosa induce interferon at 25% level of induction of a referent interferon inducer.

### Example 5.

Determination of apoptotic and apoptosis-modulating activity of the BAS.

It is known that flavonoid compounds are actively studied with reference to induction of apoptosis and the effect on cell differentiation, since they have the selective effect on leucosis and normal cells.

An investigation of BAS cytotoxicity, its ability to induce apoptosis and interaction of the BAS with cytotoxic apoptosis-inducing drug Vepesid in the system of malignant lymphoid human cells Jurkat was performed.

Investigations were performed in passaged Jurkat cell line of T-cell lymphoblastic human leucosis. Cells were cultivated with the use of common method. For the studies, the BAS was applied into the cultures at the beginning of logarithmic phase of their growth.

For analysis of cytotoxicity, apoptosis induction and distribution in the cycle phases, the BAS was introduced into the cultural medium of culture suspensions in concentration mentioned below in the text. Vitality of the cells was determined by trypan blue staining. After the end of incubation with corresponding compounds cyto-centrifuged cell samples were Pappenheim stained.

Before cytometric analysis, the cells were incubated in propidium iodide (50 mg/ml) with 0.1% sodium citrate and 0.1% triton X-100. The cell fluorescence was assessed with use of a flow cytofluorimeter FACScan of Becton Dickinson (USA). Apoptotic index (AI) was calculated with use of formula: (amount of apoptotic cells/entire amount of cells) × 100.

In cells, which were stained with propidium iodide, distribution by cell cycle phases was studied, using flow cytometry.

The results of flow cytometry were assessed with use of computer programs Mod Fit LT 2.0 (Verify Software House, Topsham, ME, USA) and CELLOQuest (BD Biosciences Pharmingen).

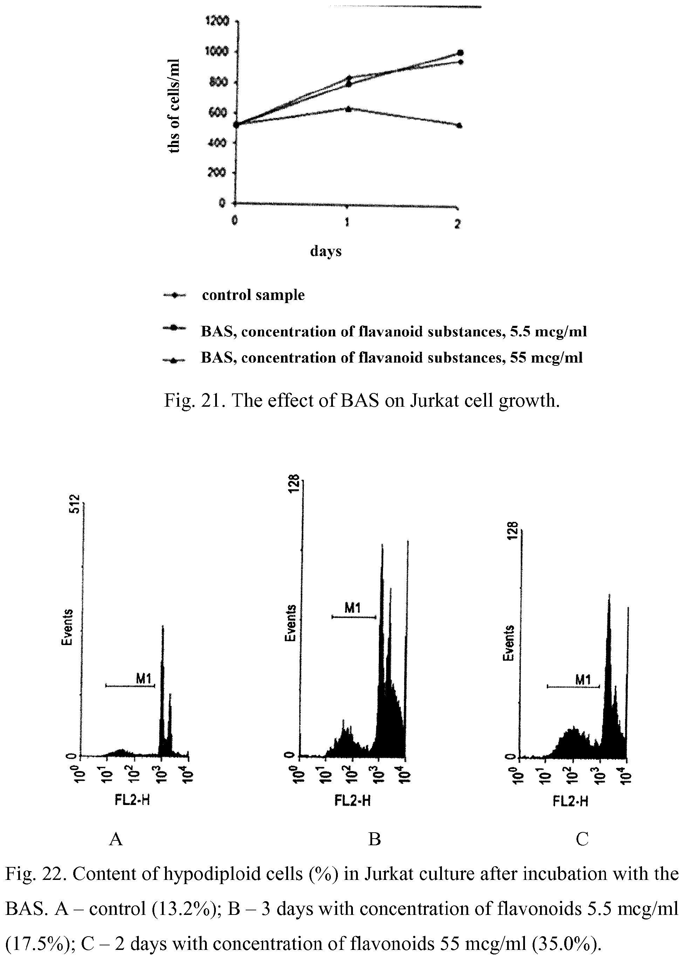

The effect of flavonoid compounds of the crude extract on Jurkat cell growth was studied for concentrations of 5.0 and 55 mcg/ml. Results are provided in Fig. 21.

As is seen from kinetics growth data, the investigational compound has low toxicity. These data coincide with relative untoxicity of extracts in other cells, both suspension and monolayer cultures of cells, which were used for examination of antiviral activity.

It should be noted that significant inhibition of cell growth under the influence of flavonoid compounds of the extract in concentration of 55 mcg/ml normally was not accompanied with massive cell death, even under complete setback of further growth.

Determination of the apoptotic components input into the effects of inhibition of cell growth was advantageous. As is seen from data provided in Fig. 22, the continuous (3 days), cultivating with extract of flavonoid compounds, even in 5.5 mcg/ml, is followed by insignificant increase of percent of hypodiploid cells in population, per flow cytometry data (in addition to spontaneous apoptosis, observed in control cultures at late stages of cultivating). Content of hypodiploid cells significantly increases with 10 time increase of flavonoid compounds concentration. On the second day, the amount of apoptotic cells increases by 20% in comparison with spontaneous apoptosis.

The obtained data show that the BAS causes a pro-apoptotic effect and in case of increasing concentration - an apoptotic one.

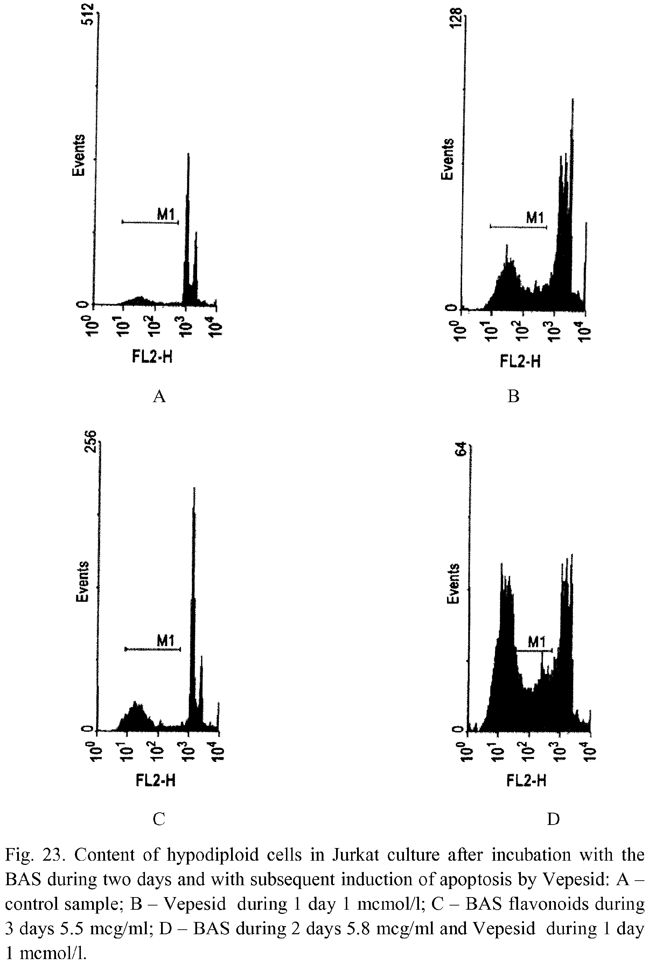

For determination of possible modifying action of flavonoid compounds on induction of apoptosis in Jurkat cells caused by Vepesid, the cells were firstly incubated with the BAS, and then after 2 or 3 days, Vepesid was applied to the cells in dose of 1 mcmol/l per day, which means that Vepesid was enough, but not excessive, for apoptosis induction. Results are provided in Fig. 23.

Thus, incubating with the BAS in non-toxic doses causes significant enhance of Jurkat cells sensitivity to typical apoptosis inducer Vepesid. Actually, in such conditions one can reach the additive effect in reference of outcome of hypodiploid cells in consecutive action of the BAS and Vepesid.

Provided results of studies show that the BAS has an apoptosis-modulating effect.

### Example 6.

Determination of the effect of the BAS on antioxidant cell status.

Results of well known studies, that referred to cytological and pharmacological effects of flavonoids in different experimental models, show that one of the mechanisms of biological action can refer to their effect on antioxidant system of cells and organism, particularly, on intensity of formation and elimination of radical oxygen forms.

Cultures of passaged lines of malignant lymphoid human cells Namalwa and MT-4 were used as an object of investigation, they were incubated with the drug during 2, 4 or 24 hours before sample collection. The BAS concentration was chosen during antiviral study and in majority of experiments it was 2.75 mcg/ml by quercetin glycoside. After the end of incubation with the BAS, the vitality of cells was controlled by staining of samples with trypan blue. For further experiments related to analysis of antioxidant properties of the BAS, only those samples, where vitality of cells after the end of incubation made not less than 90%, were used. For investigation of the drug effect on pro-antioxidant status of examined cells, we used method of EPR-spectrometry (electronic paramagnet resonance spectrometry) and chemoluminescence.

Speed of generation of superoxide radical was determined in an EPR-spectrometer with the use of spin trap 2,2,6,6-tetramethyl piperidine-N-oxide (TEMPO), that forms a stable TEMPO-radical with short-living superoxide. Reaction of accumulation of stable TEMPO-radicals in an EPR spectrometer cuvette after inctroduction of the trap into the cell culture processed linearly during 5-6 minutes. Spectra were registered with minute intervals. Sizes of amplitudes of the second component of EPR TEMPO-radical spectrum allowed to register the speed of accumulation of TEMPO-radical, that correlates with the speed of generation of a superoxide anion radical.

Working volume of the cuvette was 170 mcl. Studies were carried out under room temperature. The cuvette was filled with either native (whole) cells or homogenized ones in the ice water bath directly before introduction into a spectrometer cuvette. The cells, either previously warmed up (5 minutes at 60 °C), or after exposure under 0-10 °C for inactivation or inhibition of enzymes, that take part in electron transfer chain, were additionally examined. All solutions were prepared ex tempore from re-crystallized reagents p.a. (pure for analysis) and distilled water.

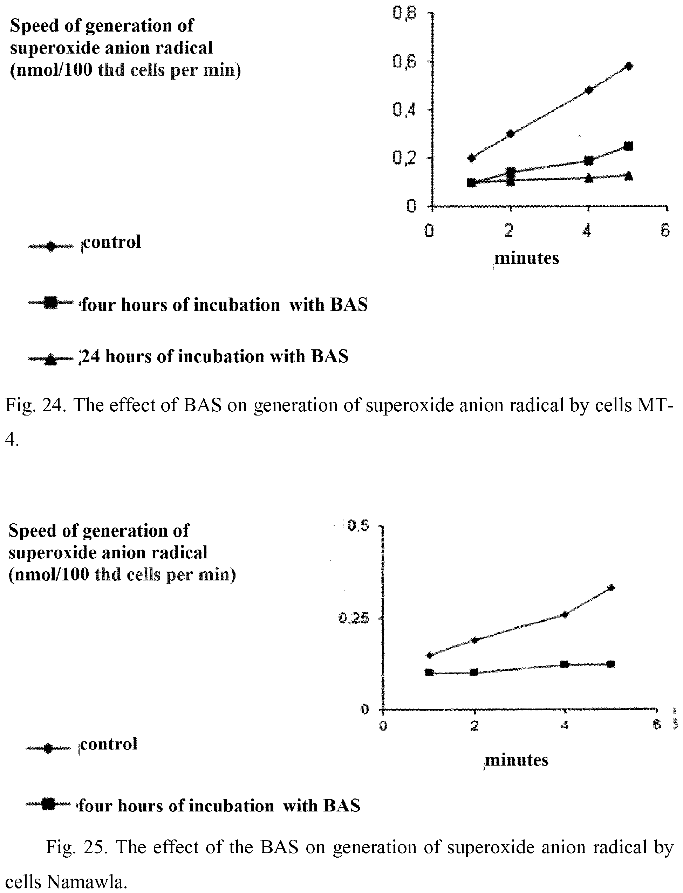

Native cells of control culture MT- generated superoxide radical anion under room temperature with an average speed as 0.53 nmol/100 thousands cells per minute (Fig. 24). After temperature processing (60 °C and 0 °C) EPR signal of TEMPO complex formation was absent, which is the evidence of participation of thermo-unstable components in regeneration of active oxygen and indicates the absence of impact of non-registered substances on the experiment results.

During the incubation of cells MT-4 in presence of the BAS, the speed of generation of superoxide radical anion decreased by 20-30% after 2 hours of incubating, and more than by half after 4 hours of incubating. After 24 hours of incubation, speed of generation of this radical was near to zero.

Activity of generation of this radical by the cells of Namalwa line was slightly below than by the MT-4 cells (in average 0.33 nmol/100 thousands cells per 1 min). Moreover, we registered actually complete inhibition of generation of radical in these cells after incubation with the BAS during all periods of observation (Fig. 25).

Performed experiments for the BAS show the presence of antioxidant properties, in particular, the dose-dependent inhibition kinetics for generation of superoxide radical anion was confirmed, as well as inhibition of intensity of free radical processes induced by hydrogen peroxide.

Examination of the BAS effect on intensity of free radical processes induced by hydrogen peroxide (H₂O₂) was performed.

Intensity of free radical processes was investigated with the use of chemoluminescence method under the room temperature. Hydrogen peroxide was used as inducer of generation of radical oxygen forms, and was introduced into a well of chemo-luminometer in an amount of 0.5 ml of 3% H₂O₂ per 1 ml of suspension of cells after prior registration of basic level of cell luminescence in open shuttle of a photomultiplier detector.

Cells, washed out from cultural medium, were introduced in concentration from 1 to 8 mln/ml.

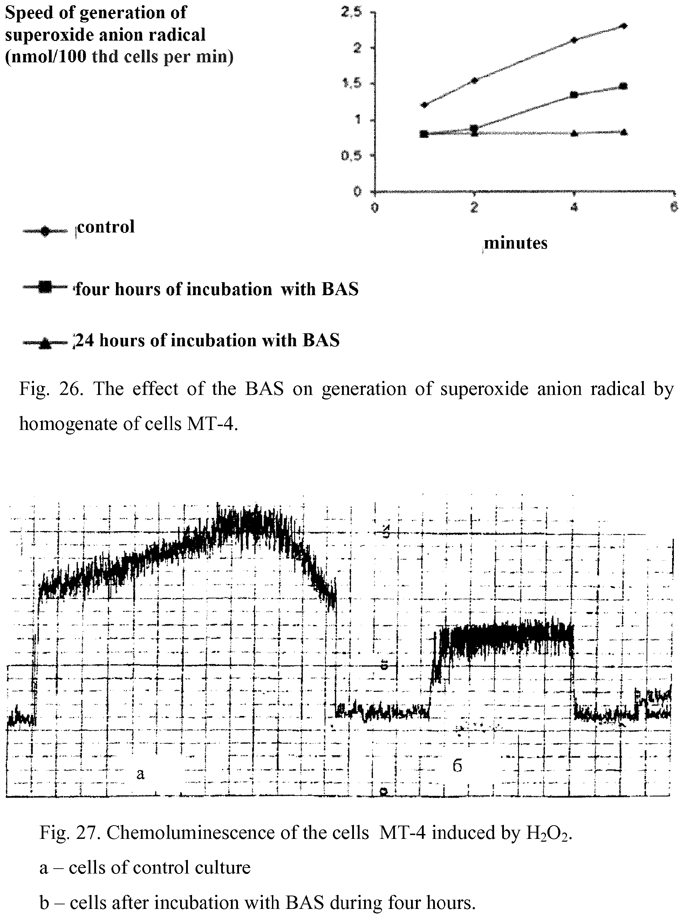

Incubation of cells MT-4 with the BAS significantly changed intensity of chemoluminescence of the cells which were attacked by H₂O₂. Typical chemo-luminograms are provided in Fig. 27. For cell samples which were incubated in presence of the BAS, the 2.2 times decrease of chemoluminescence during the first seconds after attack by H₂O₂ appeared to be characteristic, in comparison with luminescence of the cells in control culture. Maximal luminescence of control cells was registered at 90-180 seconds, after which reaction proceeded into steady state phase with luminescence amplitude 62-75 c.u. (conditional units). In cell samples, incubated with the BAS, the development of free radical reactions was sluggish - maximal luminescence appeared at 270-300 seconds, and the steady state level - at 560-600 seconds (Fig. 26).

It was found that the studied BAS has antiradical properties, namely increases resistance of the cells to free radical stress, caused by H₂O₂.

Provided examples of specific action of the BAS show that the BAS is a compound of a polypharmacologic effect. Biological spectrum of the action of the BAS, obtained from mixture of herbal extracts of Calamagrostis epigejos and Deschampsia cespitosa (1:1) and spectrum of action of the BAS, obtained from each herb separately, is the same and depends only on concentration of compounds in conversion to cumulative amount of flavonoid compounds.

Thus, the improvement of the BAS with determination of specific compositions of active ingredients of the BAS, their physical, chemical, and biological characteristics leads to invention of its optimal composition having an antiviral effect against certain viruses and dosages when creating its medicinal forms. In addition, it has been determined that the BAS is an inducer of a type-γ endogenous interferon, displays an apoptosis-modulating effect, has antioxidant properties and enhances cell resistance to free radical stress. An antiviral effect with regard to specific viruses has been established to be an antiviral effect with regard to the type 2 herpes simplex virus, the influenza virus, and the bovine viral diarrhea virus (hepatitis C virus).

Compounds that are contained in the BAS are able to block development of both DNA and RNA-containing viruses.

Studied extracts effectively inhibit the type 2 herpes simplex virus. Minimal active concentration when the virus specific action is inhibited by 2.0-3.0 1g TCD₅₀, for the extract of Deschampsia cespitosa was 0.017 mcg/ml which is twice less than the value of minimal active concentration (0.034 mcg/ml) for the crude extract and the extract of Calamagrostis epigejos. CTI of all extracts made approximately 160.

All model extracts are effective against the influenza virus. The infectious titer of the influenza virus decreases mostly (by 3.0 1g ID₅₀) when the concentration of the BAS is equal to 0.0034 mcg/ml for the extract of Deschampsia cespitosa. The most selective effect on the influenza virus is attributable to the crude extract, for which CTI=808. Results of investigations show that model extracts are effective inhibitors of the surrogate hepatitis C virus (VBVD) with high values of CTI.

All extracts inhibit the bovine diarrhea virus (test-model for hepatitis C virus) by 4.0 -5.0 1g ID₅₀ when concentrations of the BAS equals to 0.0034 mcg/ml.

BAS concentration for obtaining of an antiviral effect of 0.017÷0.068 mcg/ml is significantly less than that of synthetic substances.

All extracts are able to induce the immune γ-interferon. The extract of Calamagrostis epigejos induces the interferon at the level of the referent inducer Poly I:Poly C, whereas the crude extract and the extract of Deschampsia cespitosa induce interferon at the level of 25% from interferon induction by the referent inducer.

The provided results of studies show that the BAS has an apoptosis modulating effect.

Performed studies of the BAS show that it has antioxidant properties, in particular, dose dependent kinetics of inhibition of generation of superoxide anion and the inhibition of intensity of free radical processes induced by hydrogen peroxide were confirmed.

Moreover, the claimed BAS does not have disadvantages, which are characteristic to synthetic drugs, provides the possibility of development of pharmaceutical formulations with precise dosages and targeted pharmaco-dynamic effect.

## Claims

1. Biologically active substance having a polypharmacological effect which is made from the green parts and spikelets of cereals of the Gramineae family, genus Calamagrostis Adans and/or genus Deschampsia Beauv, and contains flavonoids, specifically aglycones of tricin, apigenin, luteolin, quercetin and rhamnazin flavonoids and/or flavonoid glycosides of tricin, apigenin, luteolin, quercetin, and rhamnazin, and excipients, and has the following composition by mass percent:
tricin flavonoid aglycone and/or tricin flavonoid glycosides: 0.016-2.062%;
apigenin flavonoid aglycone and/or apigenin flavonoid glycosides: 0.010-1.393%;
luteolin flavonoid aglycone and/or luteolin flavonoid glycosides: 0.01-4.979%;
quercetin flavonoid aglycone and/or quercetin flavonoid glycosides:
0.001-0.771%;
rhamnazin flavonoid aglycone and/or rhamnazin flavonoid glycosides:
0.104-0.203%;
excipients: 99.868-90.592%.

2. The biologically active substance according to claim 1 characterized with that it contains hydrocarbon compounds, amino acids, chlorophylls as excipients.

3. The biologically active substance according to claims 1 or 2 characterized with that it is made from the wood small-reed (Calamagrostis epigejos L. genus Calamagrostis Adans) and/or the tufted hairgrass (Deschampsia cespitosa L. genus Deschampsia Beauv).

4. The biologically active substance according to any of the preceeding claims characterized with that the content of O-glycoside forms of flavonoids is (53.545÷86.422)% and the content of C-glycoside form of flavonoids is (43.293÷4.910)% of total flavonoid compounds in the biologically active substance, the rest are flavonoid aglycones.
